# EUROPÄISCHE PATENTSCHRIFT

(11) **EP 0 700 392 B1**
(45) Veröffentlichungstag und Bekanntmachung des Hinweises auf die Patenterteilung: **09.12.1998**
(21) Anmeldenummer: 94915565.9
(22) Anmeldetag: 29.04.1994
(51) Int. Cl.: C07D 257/02, A61K 49/00, A61K 49/04

(54) **VERWENDUNG VON MAKROCYCLISCHEN METALLKOMPLEXEN ALS TEMPERATURSONDEN**
USE OF MACROCYCLIC METAL COMPLEXES AS TEMPERATURE PROBES
UTILISATION DE COMPLEXES METALLIQUES MACROCYCLIQUES COMME SONDES THERMIQUES

(30) Priorität: 28.05.1993 DE 4318369
(43) Veröffentlichungstag der Anmeldung: 13.03.1996
(73) Patentinhaber: SCHERING AKTIENGESELLSCHAFT, 13342 Berlin (DE)
(72) Erfinder: PLATZEK, Johannes, D-14195 Berlin (DE); RADÜCHEL, Bernd, D-13465 Berlin (DE); NIEDBALLA, Ulrich, D-14195 Berlin (DE); WEINMANN, Hanns-Joachim, D-14129 Berlin (DE); BAUER, Hans, D-12107 Berlin (DE); ROTH, Klaus, D-14167 Berlin (DE)
(86) Internationale Anmeldenummer: EP9401376
(87) Internationale Veröffentlichungsnummer: WO9427977

(56) Entgegenhaltungen:
- EP-A- 0 255 471
- EP-A- 0 292 689
- EP-A- 0 299 795
- EP-A- 0 434 345
- EP-A- 0 434 346
- EP-A- 0 448 191
- EP-A- 0 485 045
- EP-A- 0 512 661
- WO-A-93/24469

## Beschreibung

Die Erfindung betrifft den in den Patentansprüchen gekennzeichneten Gegenstand, das heißt, die Verwendung von makrocyclischen Metallkomplexen als Temperatursonden in der NMR-Diagnostik.

Mit Hilfe moderner diagnostischer Methoden gelingt es kleinste morphologische Strukturen mit einer Auflösung darzustellen, die der von Gewebeschnitten aus Anatomielehrbüchern nahekommt. Diese ernorm hohe Auflösung wird zum einen durch eine ständig verbesserte Gerätetechnik, zum anderen aber auch durch den Einsatz von Kontrastmitteln erreicht. Es gelingt jedoch mit den verschiedenen bekannten Methoden, wie Ultraschall-, der Röntgen-Diagnostik, der Nuklearmedizin und selbst der Kernspintomographie nicht, Informationen über den stoffwechselphysiologischen Zustand eines Gewebes des lebenden Organismus zu erhalten. Für eine genauere Diagnose und insbesondere für eine Planung und Verlaufskontrolle eines therapeutischen Einsatzes ist jedoch diese Kenntnis von erheblicher Bedeutung, da eine optimale Therapie nur dann erfolgreich sein kann, wenn frühzeitig eine Aussage über deren Wirkung möglich ist.

Es ist bekannt, daß ein wichtiger Faktor der stoffwechselphysiologischen Aktivität die Temperatur ist. Die Bestimmung dieser Gewebe-Temperatur führt zu wichtigen Aussagen über Funktion und Zustand der Zellen, so daß es wünschenswert ist, Orte zu lokalisieren, die eine von der Normalkörpertemperatur abweichende Temperatur haben. Dadurch ist es möglich, pathologisch verändertes Gewebe zu identifizieren und ggf. eine Therapie vorzunehmen.

Die Körpertemperatur ist ein Produkt der Aktivität des Energiestoffwechsels und unterliegt vielfältigen Einflüssen.

Der Blutfluß stellt eine wesentliche Einflußgröße der lokalen Gewebetemperatur dar, durch den der Körper versucht, ständig auftretende Temperaturgefälle auszugleichen [K. Brück, Wärmehaushalt und Temperaturregelung, in: Physiologie des Menschen, R.F. Schmidt G. Thews (Hersg.), Springer Verlag, 23. Auflage, 1987]. Die Temperaturmessung bietet daher eine Möglichkeit, lokale Mehr- (z.B. bei Entzündungen) oder Minderdurchblutungen (z.B. in ischämischen Gebieten) eines Gewebes gegen seine gesunde Umgebung abzugrenzen.

Bei der Hyperthermiebehandlung von Tumorerkrankungen ist die Messung der Gewebetemperatur eine wichtige Kenngröße zur Verlaufskontrolle der Bestrahlung. Bislang können dafür nur invasive Methoden genutzt werden [P. Fessenden, Direct Temperature Measurement, in: Hyperthermia in Cancer Treatment, Cancer Research, 44 (Suppl.), 4703s-4709s, 1984].

Es ist nun bekannt, daß die chemische Verschiebung von Signalen in der in-vitro NMR-Spektroskopie auch eine Funktion der Temperatur ist. Dieser Einfluß wird durch inter- und intramolekulare Wechselwirkungen hervorgerufen. In der hochauflösenden NMR Spektroskopie bestimmen intermolekulare Wechselwirkungen z.B. mit dem Lösungsmittel ganz entscheidend die chemische Verschiebung. Die Solvatation des untersuchten Moleküls, einschließlich zwischenmolekularer Aggregation und Wasserstoffbrückenbildung, ist stark von der Temperatur abhängig. Wasserstoffbrückenbindungen werden bei höheren Temperaturen aufgebrochen und ändern damit die chemische Umgebung der Atomkerne. Bei Substanzen, die starke intermolekulare Wasserstoffbrückenbindungen ausbilden, ist der Temperaturkoeffizient der chemischen Verschiebung besonders groß. Mit Hilfe von Eichkurven kann dann die Temperatur aus der experimentell gemessenen chemischen Verschiebung genau bestimmt werden. Bewährt haben sich hierbei vor allem die zu starken Wasserstoffbrückenbindungen neigenden aliphatischen Alkohole:
- Methanol CH₃OH:: T = 409,0- 36,54 Δδ - 21,85 (Δδ)²
- Ethylenglykol HOCH₂-CH₂OH:: T = 466,5- 102,00 Δδ
wobei Δδ die Differenz der chemischen Verschiebungen zwischen OH- und CH-Signal in ppm und T die absolute Temperatur in K ist [R. Duerst, A. Merbach, Rev. Sci. Instrum. 36, 1896 (1965)].

Die Änderung der chemischen Verschiebung mit der Temperatur durch intermolekulare Wechselwirkung ist keineswegs auf das Proton beschränkt, sondern eine allgemeine Eigenschaft aller magnetisch aktiven Atomkerne, so daß eine ganze Anzahl von Temperaturstandards in der Literatur vorgeschlagen wurden.

| **Standardsubstanz** | **Atomkern** | **Temp.-gradient ppm/K** | **Literatur*** |
|---|---|---|---|
| Methanol | Proton | 0,015 | (1) |
| Ethylenglykol | Proton | 0,016 | (1), (9) |
| CH₂I₂/Cyclooctan | Kohlenstoff | 0,07 | (2) |
| CH₃I/Tetramethylsilan | Kohlenstoff | 0,02 | (2) |
| MgATP-Komplex | Phosphor | 0,012 | (3), (9) |
| K₃Co(CN)₆ | Kobalt | 1,504 | (4), (9) |
| Co-acetylacetonat | Kobalt | 3,153 | (4), (9) |
| (CBrF₂)₂/CClF2)₂ | Fluor | 0,0071 | (5) |
| CFCl₃/CBr₂F₂ | Fluor | 0,0012 | (5) |
| Perfluortributylamin | Fluor | 0,0003 | (6) |
| (CH₃)₂Tl NO₃ | Thallium | 0,44 | (7) |
| Methanol | Deuterium | 0,015 | (8) |

| | | | |
|---|---|---|---|
| *siehe Literaturverzeichnis | | | |

Weiterhin wurde ein ¹³C-NMR-Thermometer vorgeschlagen, das auf der Änderung der Komplexbildungskonstante zwischen dem Verschiebungsreagenz Yb(fod)₃ und Aceton basiert [H.J. Schneider, W. Freitag, M. Schommer, J. Magn. Reson. 18, 393 (1973)]. Dieses Verfahren kann allerdings nur in organischen Lösemitteln angewendet werden.

Der Einfluß von intramolekularen Wechselwirkungen auf die chemische Verschiebung ist bei praktisch allen organischen Verbindungen zu gering, um für Temperaturmessungen herangezogen zu werden. In der Literatur ist nur ein Beispiel dieser Art beschrieben, bei dem die intramolekulare Rotationsbarriere im Furfural und die damit verbundene Linienformveränderungen im ¹³C-NMR-Spekrum zur Temperaturmessung benutzt wurde [S. Combrisson, T. Prange, J. Magn. Reson. 19, 108 (1973)]. Dieses Verfahren eignet sich aber nur in einem sehr schmalen Temperaturbereich und dieser Meßbereich hängt stark von der verwendeten magnetischen Meßfeldstärke ab.

Eine Anwendung dieser Methode zur in-vivo Temperaturmessung von Körpergewebe scheiterte jedoch bislang aus vielfältigen Gründen. So sind die meisten der in der Literatur beschriebenen Verbindungen mit Wasser nicht mischbar, bzw. nur in unpolaren organischen Lösemitteln wie Chloroform löslich. Damit ist ein Einsatz in intakten biologischen Systemen praktisch ausgeschlossen. Zwar gelang die Einbringung einer reinen Perfluortributylaminblase in ein Kaninchenauge und die anschließende Temperaturmessung [B. A. Berkowitz, J.T. Handa, C.A. Wilson, NMR in Biomedicine 5, 65 (1992)], jedoch ist dieses Verfahren hochinvasiv und nicht auf andere Organe übertragbar. Einige der wasserlöslichen Verbindungen wie Methanol, Ethylenglykol, K₃Co(CN)₆ und Thalliumsalze müssen aufgrund der hohen Toxizität von vornherein ausgeschlossen werden. Von den beschriebenen Substanzen ist theoretisch allein der MgATP-Komplex als körpereigene Temperatursonde geeignet. Da die relative MgATP-Konzentration im Cytosol gering ist (um 10 mmol/kg) und wegen des Kreatininkinase-Gleichgewichts durch externe Zugabe nicht erhöht werden kann und weiterhin der Phosphorkern unempfindlich und die chemischen Verschiebungen zusätzlich stark von der Ionenstärke und dem pH des Mediums abhängig sind, ist eine präzise Temperaturbestimmung mit ³¹P-NMR-Messungen am MgATP in vertretbarer Zeit nicht möglich. Somit sind alle in der Literatur beschriebenen Temperatursonden für eine in vivo Temperaturmessung in der klinischen Routine-Diagnostik ungeeignet.

Aufgabe der vorliegenden Erfindung war es daher geeignete Verbindungen für die in-vivo Temperaturmessung mittels NMR-Spektroskopie zu finden.
Diese Verbindungen müssen die folgenden Anforderungen erfüllen:
a) Sie müssen auf eine Veränderung der Temperatur mit einer veränderten Resonanzfrequenz im NMR-Spektrum reagieren,
b) eine starke chemische Verschiebung pro Grad-Temperaturänderung,
c) eine für diagnostische Anwendungen geeignete Pharmakokinetik,
d) eine für eine Messung genügend hohe Anreicherung in den Zielgeweben,
e) eine gute Verträglichkeit und geringe Toxizität,
f) eine metabolische Stabilität,
g) eine hohe chemische Stabilität und Lagerfähigkeit und
h) gute Wasserlöslichkeit aufweisen

Es wurde nun gefunden, daß makrocyclische Metallkomplexe bestehend aus mindestens einem Metallion eines Elementes der Ordnungszahlen 21-29, 42, 44 oder 57-70 und einem Komplexbildner der allgemeinen Formel I 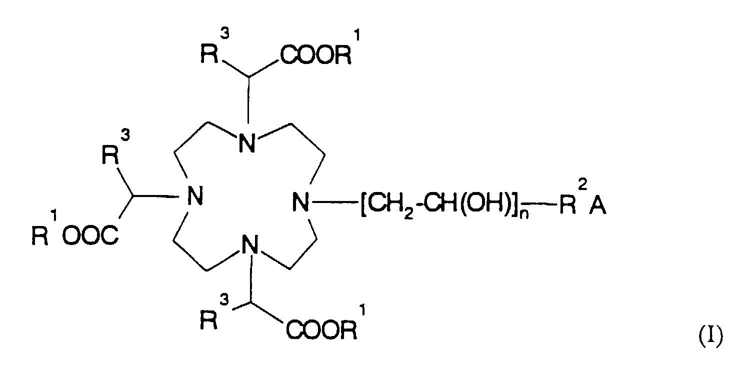 worin
- n: die Ziffern 0 oder 1 bedeutet
- R¹: unabhängig voneinander für ein Wasserstoffatom oder ein Metallionenäquivalent,
- R³: für ein Wasserstoffatom, eine geradkettige oder verzweigte C₁-C₁₀ Alkylgruppe, die gegebenenfalls durch 1-5 C₁-C₆-Alkoxygruppen, Hydroxy-C₁-C₆-alkylgruppen und/oder Hydroxygruppen substituiert ist,
- R²: eine geradkettige oder verzweigte C₁-C₁₀ Alkylengruppe, die gegebenenfalls durch 1 bis 5 Sauerstoffatome und/oder Carbonylgruppen unterbrochen ist und/oder gegebenfalls durch 1 bis 5 Hydroxygruppen, C₁-C₆-Alkoxy-C₁-C₆-alkylgruppen, -OR⁴ -, -CO-NR⁵R⁶ -, -NR⁵R⁶ - und/oder -NR⁵-CO-R⁶- Reste substituiert ist, worin R⁴ für einen geradkettigen oder verzweigten C₁-C₄ Alkylrest steht und R⁵, R⁶ unabhängig voneinander die Bedeutung von R³ haben und
- A: für ein Wasserstoffatom oder einen zweiten makrocyclischen Rest der allgemeinen Formel II steht, 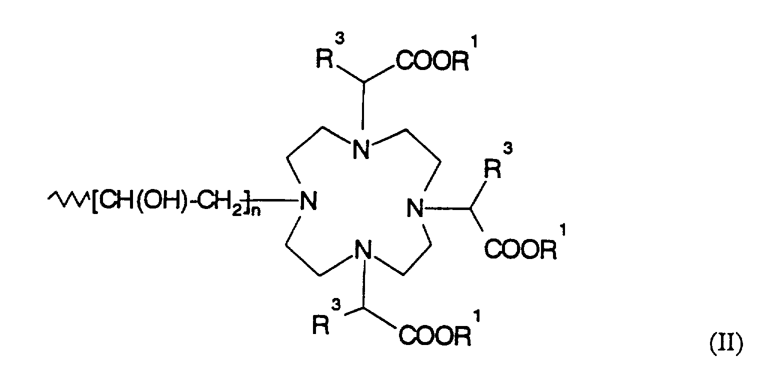
worin
n, R¹ und R³ die angegebenen Bedeutungen haben,
wobei freie nicht zur Komplexierung der Metallionen benötigten Carbonsäuregruppen gewünschtenfalls als Salz einer anorganischen oder organischen Base oder Aminosäure und/oder als Ester oder Amid vorliegen, wobei mindestens zwei Reste R¹ für ein Metallionenäquivalent stehen, als Temperatursonden in der NMR-Diagnostik geeignet sind.

Bevorzugt sind Verbindungen bei denen R³ für ein Wasserstoffatom und R²-A für eine -CH₂CH₂OCH₃-, -CH₂CH₂O-C(CH₃)₃-, -CH₂-CH(OH)-CH₂OCH₃-, -CH₂CH(OH)-CH₂O-CH(CH₃)₂-, -CH₂-CH(OH)-CH₂O-C(CH₃)₃ -, -CH₂-CH(OH)-CH₃-, -CH(CH₂OCH₃)₂-, -CH(CH₂OCH₃)-CH(OH)CH₂OH-, -CH₂-CH₂-NH-CH₃-, -CH₂-CH₂-N(CH₃)₂-, -CH₂-CO-N(CH₃)₂-, -CH₂-CH₂-O-CH₂-CH₂-O-CH₃-, -C(CH₂OCH₃)₃ -Gruppe steht.

Besonders bevorzugt sind aus dieser Gruppe die Reste R²-A, wie z.B. -CH₂CH₂OCH₃-, -CH₂CH₂O-C(CH₃)₃, da diese eine geringere Linienbreite aufweisen, und daher die Änderung der chemischen Verschiebung präziser bestimmt werden kann.

Überraschend ist, daß die chemischen Verschiebungsänderungen der genannten Komplexverbindungen bei geringen Konzentrationen, wie sie für die in-vivo Anwendung gebraucht werden, im wesentlichen intramolekularen Ursprungs und dadurch unabhängig von externen Einflüssen wie Ionenstärke, pH und Sauerstoffpartialdruck sind, d.h. die Temperaturabhängigkeit der chemischen Verschiebung beruht allein auf der Wechselwirkung zwischen einem Zentralion und den sich im Liganden befindenen Atomkernen.

Überraschend ist weiterhin, daß diese durch intramolekulare Wechselwirkung hervorgerufene chemische Verschiebung ausreichend groß ist, um für eine in-vivo Temperaturmessung herangezogen werden zu können.

Die Temperaturgradienten sind vom gemessenen Atomkern, der chemischen Struktur des Liganden und dem Zentralion abhängig.

Als Zentralatom besonders geeignet sind paramagnetische Metallionen, insbesondere die der Lanthanoid-Elemente.

Überraschenderweise wurde gefunden, daß der Temperatureinfluß auf die chemische Verschiebung der durch die Komplexe hervorgerufenen Signale nicht für alle Komplexe in die gleiche Richtung verläuft. So wird z.B. das Methoxygruppensignal des Europium-Komplexes (hergestellt nach Beispiel 6h) nach tiefem und das des entsprechenden Praseodym-Komplexes (hergestellt nach Beispiel 6c) nach höherem Feld verschoben, d.h. der Praseodym-Komplex weist einen positiven Temperaturgradienten auf, wohingegen der Europium-Komplex einen negativen Temperaturgradienten zeigt. Damit ergibt sich die Möglichkeit, durch Mischung der beiden Komplexe in den diagnostischen Mitteln die Präzision der Temperaturmessung deutlich zu erhöhen.

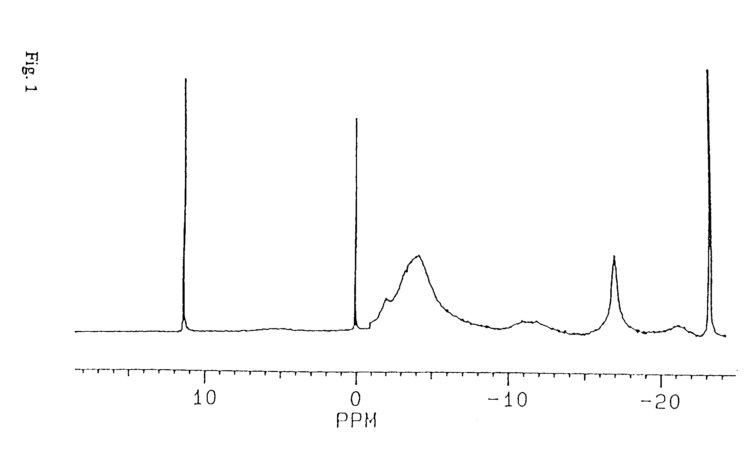

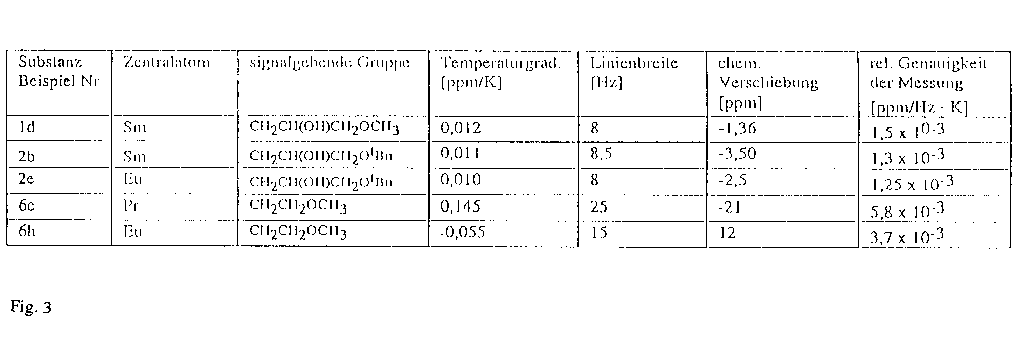

Fig. 1/3 zeigt eine Superposition der bei Raumtemperatur aufgenommenen in-vitro Spektren des genannten Europium- und Praseodymkomplexes in D₂O. Das Singulett bei - 23 ppm entspricht der Methoxygruppe des Praseodymkomplexes, das Singulett bei 11,2 ppm entspricht der Methoxygruppe des Europiumkomplexes. Als interner Standard wurde das Signal des D₂O verwendet. Zur Bestimmung der Temperaturgradienten wurde eine 0,01 molare Lösung der betreffenden Komplexe in D₂O hergestellt. Von den jeweiligen Lösungen wurden Spektren bei verschiedenen Temperaturen im Bereich zwischen 46 °C und 26 °C aufgenommen. Aus diesen Messungen ergibt sich für den Praseodymkomplex (hergestellt nach Beispiel 6c) ein Temperaturgradient von 0,145 ppm/K.

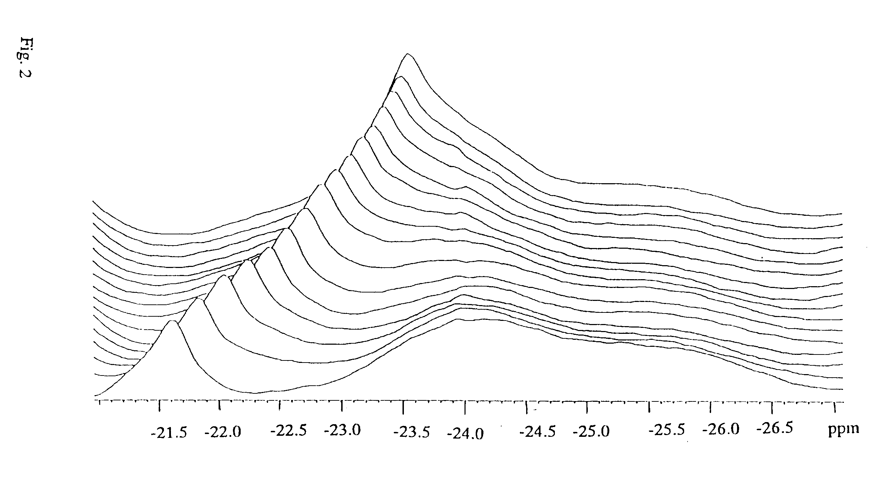

Fig. 2/3 zeigt die zeitliche Veränderung des NMR-Spektrums während der Abkühlung der Lösung des genannten Praseodymkomplexes. Unter Berücksichtigung der Linienbreite ergibt sich eine Messgenauigkeit von 0,3 °C.

Ein für die lokalisierte in vivo ¹H-NMR-Spektroskopie ganz entscheidender Vorteil dieser Verbindungsklasse liegt darin, daß die zur Temperaturmessung benutzten Signale außerhalb des spektralen Bereichs aller körpereigenen Substanzen einschließlich des Gewebewassers erscheinen. Dies erlaubt die nahezu störungsfreie Messung der temperaturempfindlichen Signale durch eine stark vereinfachte Unterdrückung von intensiven Hintergrundsignalen für die NMR-Spektroskopie.

Ein weiterer Vorteil bietet die große Flexibilität der chemischen Struktur des Liganden, die dem zu lösenden Meßproblem angepaßt werden kann. Durch entsprechende Einstellung der longitudinalen Relaxationszeit T₁ des Meßsignals kann z. B. eine optimale Empfindlichkeit erzielt werden. Für lokalisierte spektroskopische Methoden, die auf einem Spin-Echo beruhen, kann auf gleichem Wege die transversale Relaxationszeit T₂ optimal eingestellt werden kann.

Die Herstellung der Komplexbildner der allgemeinen Formel I (d.h. von Verbindungen bei denen R¹ für ein Wasserstoffatom steht) erfolgt wie in den europäischen Patentschriften EP 0 299 795, EP 0 434 345, EP 0 434 346 und der EP 0 255 371 beschrieben.

Verbindungen der Formel I bei denen R³ ungleich Wasserstoff ist, lassen sich wie in der DE 41 40 779 beschrieben herstellen, indem zunächst die Seitenkette -R²-A durch Umsetzung des entsprechenden Epoxids mit Tricyclotridecan eingeführt wird. Nach Abspaltung der Formylgruppe des entstanden Intermediates wird das so erhaltene Produkt mit einer Verbindung der allgemeinen Formel III umgesetzt, 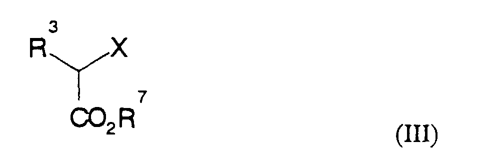 worin X für ein Nucleofug, z.B. für ein Halogen oder einen Sulfonyloxyrest und R⁷ für ein Wasserstoffatom oder für eine Säureschutzgruppe, bevorzugt für eine tert.-Butylgruppe, steht.

An Stelle des oben eingesetzten Tricyclotridecan kann auch Tris(benzyloxycarbonyl)cyclen mit Epoxiden alkyliert werden (s. J. Chem. Soc. Perkin Trans. I,12 p. 3329 [1991]).

Die für die Umsetzungen benötigten Epoxide sind nach dem Fachmann bekannten Verfahren herstellbar.

Die Umsetzung von 1,4,7-Triscarboxymethyl-1,4,7,10-tetraazacyclododecan mit Epoxiden erfolgt in Lösungsmitteln bei Temperaturen zwischen 10 und 150°C, bevorzugt bei 50-80°C. Als Lösungsmittel kommen alle inerten, polaren Lösungsmittel in Frage. Die Reaktion wird unter Zusatz von Basen ausgeführt. Die Base kann in fester oder gelöster Form zugefügt werden. Als Basen kommen in Betracht Lithiumhydroxid, Natriumhydroxid, Kaliumhydroxid, Alkali- und Erdalkalicarbonate und -oxide, oder organische Basen, wie tertiäre Amine, z.B. Triethylamin oder Diisopropylethylamin, N-Methyl-Morpholin oder Tetramethylpiperidin.

Die Umsetzung von 1,4,7-Triscarboxymethyl-1,4,7,10-tetraazacyclododecan mit Halogenverbindungen kann ebenfalls in fester oder flüssiger Form durchgeführt werden. Bevorzugte Lösungsmittel sind Dioxan, Tetrahydrofuran, Dimethylformamid, Dimethylacetamid und Methanol, Ethanol, Propanol, Isopropanol und Wasser. Die Reaktion erfolgt unter Zusatz von Basen (wie zuvor angegeben) bei Temperaturen zwischen 40°C und 150°C, bevorzugt bei 75° bis 110°C.

Die Herstellung der erfindungsgemäßen Metallkomplexe aus diesen Komplexbildnern erfolgt in der Weise, wie sie in der Deutschen Offenlegungsschrift DE 34 01 052 sowie in den europäischen Patentanmeldungen EP 0 450 742 und EP 0 413 405 offenbart worden sind, indem man das Metalloxid oder ein Metallsalz (beispielsweise das Nitrat, Acetat, Carbonat, Chlorid oder Sulfat) des Elements der Ordnungszahlen 21-29, 42, 44, 57-70 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome durch Kationen anorganischer und/oder organischer Basen oder Aminosäuren substituiert.

Die Neutralisation eventuell noch vorhandener freier Säuregruppen erfolgt mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler und saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Die erfindungsgemäßen pharmazeutischen Mittel werden vorzugsweise in einer Konzentration von 1 µmol-1 mol/l hergestellt. Sie werden in der Regel in Mengen von 0,005-20 mmol/kg Körpergewicht, vorzugsweise 0,05-5 mmol/kg Körpergewicht, dosiert. Sie sind zur enteralen und parenteralen Applikation bestimmt. Die erfindungsgemäßen Mittel erfüllen die vielfältigen Voraussetzungen für die Eignung als Diagnostika für die NMR-Spektroskopie. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen gebundenen Ionen innerhalb der Zeit, in der die neuen Kontrastmittel vollständig wieder ausgeschieden werden, nur äußerst langsam erfolgt.

Durch die Verwendung dieser sehr gut verträglichen Komplexe als neuartige Meßsonden ist es somit möglich geworden, in kleineren Volumina (z. B. 10 cm³) ortsaufgelöste Spektroskopie durchzuführen und die Temperatur präzise in kurzer Meßzeit ohne Störung bzw. Überlagerung durch andere Moleküle zu bestimmen.

Die nachfolgenden Beispiele dienen der Erläuterung des Erfindungsgegenstandes, ohne ihn auf diese beschränken zu wollen.

### Beispiel 1

a) 10-(2-Hydroxy-3-methoxy-propyl)-1,4,7-tris-carboxymethy]-1,4,7,10-tetraazacyclododecan
7,63 g (86,58 mmol) Glycidylmethylether und 10 g (28,86 mmol) 1,4,7-Triscarboxymethyl-1,4,7,10-tetraazacyclododecan werden in einer Mischung aus 50 ml Dioxan/80 ml Wasser gelöst, und der pH-Wert mit 6 N Kalilauge auf pH 10 gebracht. Man rührt 24 Stunden bei 50°C. Man dampft zur Trockne ein, nimmt den Rückstand mit 300 ml Wasser/50 ml Methanol auf und extrahiert 2 mal mit 100 ml tert.-Butyl-methylether. Die wäßrige Lösung wird mit 5 N-Salzsäure auf pH 1 gestellt und zur Trockne eingedampft. Der Rückstand wird mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/ Laufmittel= Gradient aus Wasser/Tetrahydrofuran) chromatographiert. Nach Eindampfen der Hauptfraktion erhält man 10,38 g (77 % d. Th.) eines stark hygroskopischen, glasigen Feststoffes.
Wassergehalt: 7,0 %

| Analyse (bezogen auf wasserfreie Substanz): | | | |
|---|---|---|---|
| ber. | C 49,76 | H 7,89 | N 12,89 |
| gef. | C 49,54 | H 7,98 | N 12,70 |

b) Lanthan-Komplex von 10-(2-Hydroxy-3-methoxy-propyl)-1,4,7-tris-carboxymethyl-1,4,7,10-tetraazacyclododecan
5 g (11,51 mmol) der Titelverbindung aus Beispiel 1a werden in 50 ml Wasser gelöst und 1,87 g (5,75 mmol) Lathanoxid zugegeben. Man rührt 3 Stunden bei 90°C. Die Lösung wird eine Stunde mit 2 ml saurem Ionenaustauscher (AMB 252c H⁺) und 2 ml schwach basischen Austauscher IRA 67 (OH⁻-Form) bei Raumtemperatur gerührt. Es wird vom Austauscher abfiltriert und das Filtrat gefriergetrocknet.
Ausbeute: 6,56 g (95 % d. Th.) eines glasigen Feststoffes
Wassergehalt: 4,9 %

| Analyse (bezogen auf wasserfreie Substanz): | | | | |
|---|---|---|---|---|
| ber. | C 37,90 | H 5,48 | N 9,82 | La 24,35 |
| gef. | C 37,79 | H 5,58 | N 9,71 | La 24,22 |

c) Praseodym-Komplex von 10-(2-Hydroxy-3-methoxy-propyl)-1,4,7-tris-carboxymethyl-1,4,7,10-tetraazacyclododecan
5 g (11,51 mmol) der Titelverbindung aus Beispiel 1a werden in 50 ml Wasser gelöst und 3,66 g (11,51 mmol) Praseodym(III)acetat zugesetzt. Man erwärmt 2 Stunden auf 90°C und dampft anschließend im Vakuum zur Trockne ein. Der Rückstand wird in 50 ml Wasser aufgenommen und erneut zur Trockne eingedampft. Der Rückstand wird in 100 ml Wasser gelöst und 1 Stunde mit 2 ml saurem Ionenaustauscher (AMB 252c/H⁺-Form) und 5 ml schwach basischem Austauscher (IRA 67/OH⁻-Form) gerührt. Es wird vom Austauscher abfiltriert und das Filtrat gefriergetrocknet.
Ausbeute: 6,75 g (96 % d. Th.) eines hellgrünen Feststoffs
Wassergehalt: 6,3 %

| Analyse (bezogen auf wasserfreie Substanz): | | | | |
|---|---|---|---|---|
| ber. | C 37,77 | H 5,46 | N 9,79 | Pr 24,62 |
| gef. | C 37,56 | H 5,69 | N 9,61 | Pr 24,48 |

d) Samarium-Komplex von 10-(2-Hydroxy-3-methoxy-propyl)-1,4,7-tris-carboxymethyl-1,4,7,10-tetraazacyclododecan
Analog zu Beispiel 1b wurde Samariumoxid anstelle von Lanthanoxid eingesetzt.
Ausbeute: 95 % d. Th. eines farblosen Feststoffs
Wassergehalt: 6,9 %

| Analyse (bezogen auf wasserfreie Substanz): | | | | |
|---|---|---|---|---|
| ber. | C 37,16 | H 5,37 | N 9,63 | Sm 25,84 |
| gef. | C 36,95 | H 5,45 | N 9,49 | Sm 25,67 |

e) Europium-Komplex von 10-(2-Hydroxy-3-methoxy-propyl)-1,4,7-tris-carboxymethyl-1,4,7,10-tetraazacyclododecan
Analog zu Beispiel 1b wurde Europiumoxid anstelle von Lanthanoxid eingesetzt.
Ausbeute: 93 % d. Th. eines amorphen Pulvers
Wassergehalt: 9,4 %

| Analyse (bezogen auf wasserfreie Substanz): | | | | |
|---|---|---|---|---|
| ber. | C 37,06 | H 5,36 | N 9,60 | Eu 26,05 |
| gef. | C 36,84 | H 5,51 | N 9,47 | Eu 25,87 |

f) Holmium-Komplex von 10-(2-Hydroxy-3-methoxy-propyl)-1,4,7-tris-carboxymethyl-1,4,7,10-tetraazacyclododecan
Analog zu Beispiel 1b wurde Holmiumoxid anstelle von Lanthanoxid eingesetzt.
Ausbeute: 97 % d. Th. eines schwach rosa fluoreszierendem Feststoffes
Wassergehalt: 7,2 %

| Analyse (bezogen auf wasserfreie Substanz): | | | | |
|---|---|---|---|---|
| ber. | C 36,25 | H 5,24 | N 9,39 | Ho 27,65 |
| gef. | C 36,13 | H 5,31 | N 9,28 | Ho 27,48 |

g) Dysprosium-Komplex von 10-(2-Hydroxy-3-methoxy-propyl)-1,4,7-tris-carboxymethyl-1,4,7,10-tetraazacyclododecan
Analog zu Beispiel 1b wurde Dysprosiumoxid anstelle von Lanthanoxid eingesetzt.
Ausbeute: 95 % d. Th. eines farblosen amorphen Pulvers
Wassergehalt: 8,1 %

| Analyse (bezogen auf wasserfreie Substanz): | | | | |
|---|---|---|---|---|
| ber. | C 36,40 | H 5,26 | N 9,43 | Dy 27,36 |
| gef. | C 36,28 | H 5,37 | N 9,31 | Dy 27,28 |

### Beispiel 2

a) 10-(2-Hydroxy-3-tert.-butyloxy-propyl)-1,4,7-tris-carboxymethyl-1,4,7,10-tetraazacyclododecan
11,27 g (86,58 mmol) Glycidyl-tert.-butylether und 10 g (28,86 mmol) 1,4,7-Triscarboxymethyl-1,4,7,10-tetraazacyclododecan werden in einer Mischung aus 50 ml Dioxan/80 ml Wasser gelöst, und der pH-Wert mit 6 N Kalilauge auf pH 10 gebracht. Man rührt 24 Stunden bei 70°C. Man dampft zur Trockne ein, nimmt den Rückstand mit 300 ml Wasser/50 ml Methanol auf und extrahiert 2 mal mit 100 ml tert.-Butyl-methylether. Die wäßrige Lösung wird mit 5 N-Salzsäure auf pH 1 gestellt und zur Trockne eingedampft. Der Rückstand wird mit 200 ml Methanol/80 ml Methylenchlorid ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP 18/ Laufmittel= Gradient aus Wasser/Tetrahydrofuran) chromatographiert. Nach Eindampfen der Hauptfraktion erhält man 10,38 g (71 % d. Th.) eines stark hygroskopischen, glasigen Feststoffes.
Wassergehalt: 5,9 %

| Analyse (bezogen auf wasserfreie Substanz): | | | |
|---|---|---|---|
| ber. | C 52,93 | H 8,46 | N 11,76 |
| gef. | C 52,80 | H 8,51 | N 11,58 |

b) Samarium-Komplex von 10-(2-Hydroxy-3-tert.-butyloxy-propyl)-1,4,7-tris-carboxymethyl-1,4,7,10-tetraazacyclododecan
5 g (10,49 mmol) der Titelverbindung aus Beispiel 2a werden in 50 ml Wasser gelöst und 1,83 g (5,24 mmol) Samariumoxid zugegeben. Man rührt 3 Stunden bei 90°C. Die Lösung wird eine Stunde mit 2 ml saurem Ionenaustauscher (AMB 252c/H⁺-Form) und 2 ml schwach basischem Austauscher IRA 67/OH⁻-Form) bei Raumtemperatur gerührt.
Ausbeute: 6,77 g (96 % d. Th.) eines glasigen Feststoffes
Wassergehalt: 7,2 %

| Analyse (bezogen auf wasserfreie Substanz): | | | | |
|---|---|---|---|---|
| ber. | C 40,43 | H 5,98 | N 8,98 | Sm 24,10 |
| gef. | C 40,21 | H 6,10 | N 8,87 | Sm 24,00 |

c) Praseodym-Komplex von 10-(2-Hydroxy-3-tert.-butyloxy-propyl)-1,4,7-tris-carboxymethyl-1,4,7,10-tetraazacyclododecan
Analog zu Beispiel 1c wurde die Titelverbindung aus Beispiel 2a anstelle der Titelverbindung aus Beispiel 1a zur Komplexierung eingesetzt.
Ausbeute: 93 % d. Th. eines grünlichen amorphen Pulvers
Wassergehalt: 5,9 %

| Analyse (bezogen auf wasserfreie Substanz): | | | | |
|---|---|---|---|---|
| gef. | C 41,05 | H 6,07 | N 9,12 | Pr 22,93 |
| ber. | C 40,90 | H 6,20 | N 9,01 | Pr 22,74 |

d) Dysprosium-Komplex von 10-(2-Hydroxy-3-tert.-butyloxy-propyl)-1,4,7-tris-carboxymethyl-1,4,7,10-tetraazacyclododecan
Analog zu Beispiel 2b wurde Dysprosiumoxid anstelle von Samariumoxid eingesetzt.
Ausbeute: 95 % d. Th. eines farblosen amorphen Pulvers
Wassergehalt: 7,4 %

| Analyse (bezogen auf wasserfreie Substanz): | | | | |
|---|---|---|---|---|
| ber. | 39,66 | H 5,86 | N 8,81 | Dy 25,55 |
| gef. | 39,49 | H 5,98 | N 8,70 | Dy 25,38 |

e) Europium-Komplex von 10-(2-Hydroxy-3-tert.-butyloxy-propyl)-1,4,7-tris-carboxymethyl-1,4,7,10-tetraazacyclododecan
Analog zu Beispiel 2b wurde Europiumoxid anstelle von Samariumoxid eingesetzt.
Ausbeute: 97 % d. Th. eines farblosen Feststoffes
Wassergehalt: 8,7 %

| Analyse (bezogen auf wasserfreie Substanz): | | | | |
|---|---|---|---|---|
| ber. | C 40,32 | H 5,92 | N 8,96 | Eu 24,29 |
| gef. | C 49,18 | H 5,99 | N 8,71 | Eu 24,05 |

f) Lanthan-Komplex von 10-(2-Hydroxy-3-tert.-butyloxy-propyl)-1,4,7-tris-carboxymethyl-1,4,7,10-tetraazacyclododecan
Analog zu Beispiel 2b wurde Lanthanoxid anstelle von Samariumoxid eingesetzt.
Ausbeute: 94 % d. Th. eines farblosen amorphen Pulvers
Wassergehalt: 8,1 %

| Analyse (bezogen auf wasserfreie Substanz): | | | | |
|---|---|---|---|---|
| ber. | C 41,18 | H 6,09 | N 9,15 | La 22,68 |
| gef. | C 41,04 | H 6,17 | N 9,02 | La 22,49 |

g) Holmium-Komplex von 10-(2-Hydroxy-3-tert.-butyloxy-propyl)-1,4,7-tris-carboxymethyl-1,4,7,10-tetraazacyclododecan
Analog zu Beispiel 2b wurde Holmiumoxid anstelle von Samariumoxid eingesetzt.
Ausbeute: 97 % d. Th. eines schwach rot fluoreszierendes Pulver
Wassergehalt: 5,9 %

| Analyse (bezogen auf wasserfreie Substanz): | | | | |
|---|---|---|---|---|
| ber. | C 39,51 | H 5,84 | N 8,78 | Ho 25,83 |
| gef. | C 39,37 | H 5,98 | N 8,60 | Ho 25,67 |

### Beispiel 3

a) Lanthan-Komplex von 10-(2-Hydroxy-propyl)-1,4,7-tris-(carboxymethyl)-1,4,7,10-tetraazacyclododecan
5 g (12,36 mmol) 10-(2-Hydroxy-propyl)-1,4,7-tris-(carboxymethyl)-1,4,7,10-tetraazacyclododecan werden in 50 ml Wasser gelöst und 2,01 g (6,18 mmol) Lanthanoxid zugegeben. Man rührt 3 Stunden bei 90°C. Die Lösung wird eine Stunde mit 2 ml saurem Ionenaustauscher (AMB 252c/H⁺-Form) und 2 ml schwach basischem Austauscher (IRA 67/OH⁻-Form) bei Raumtemperatur gerührt. Es wird vom Austauscher abfiltriert und das Filtrat gefriergetrocknet.
Ausbeute: 6,53 g (94 % d. Th.) eines glasigen Feststoffes
Wassergehalt: 3,8,%

| Analyse (bezogen auf wasserfreie Substanz): | | | | |
|---|---|---|---|---|
| ber. | C 37,79 | H 5,41 | N 10,37 | La 25,71 |
| gef. | C 37,58 | H 5,60 | N 10,18 | La 25,52 |

b) Samarium-Komplex von 10-(2-Hydroxy-propyl)-1,4,7-tris-(carboxymethyl)-1,4,7,10-tetraazacyclododecan
Analog zu Beispiel 3a wurde aus Samariumoxid und 10-(2-Hydroxy-propyl)-1,4,7-tris-(carboxymethyl)-1,4,7,10-tetraazacyclododecan die Titelverbindung 3b hergestellt.
Ausbeute: 95 % d. Th. eines farblosen Feststoffes
Wassergehalt: 6,1 %

| Analyse (bezogen auf wasserfreie Substanz): | | | | |
|---|---|---|---|---|
| ber. | C 37,00 | H 5,30 | N 10,15 | Sm 27,25 |
| gef. | C 36,88 | H 5,41 | N 10,02 | Sm 27,10 |

c) Holmium-Komplex von 10-(2-Hydroxy-propyl)-1,4,7-tris(carboxymethyl)-1,4,7,10-tetraazacyclododecan
Analog zu Beispiel 3a wurde aus Holmiumoxid und 10-(2-Hydroxy-propyl)-1,4,7-tris(carboxymethyl)-1,4,7,10-tetraazacyclododecan die Titelverbindung 7c hergestellt.
Ausbeute: 97 % d. Th. eines schwach rosafarbenen Feststoffes
Wassergehalt: 8,7 %

| Analyse (bezogen auf wasserfreie Substanz): | | | | |
|---|---|---|---|---|
| ber. | C 36,05 | H 5,16 | N 9,89 | Ho 29,12 |
| gef. | C 35,87 | H 5,29 | N 9,71 | Ho 29,01 |

d) Europium-Komplex von 10-(2-Hydroxy-propyl)-1,4,7-tris-(carboxymethyl)-1,4,7,10-tetraazacyclododecan
Analog zu Beispiel 3a wurde aus Europiumoxid und 10-(2-Hydroxy-propyl)-1,4,7-tris-(carboxymethyl)-1,4,7,10-tetraazacyclododecan die Titelverbindung 3d hergestellt.
Ausbeute: 97 % d. Th. farbloser Feststoff
Wassergehalt: 5,7 %

| Analyse (bezogen auf wasserfreie Substanz): | | | | |
|---|---|---|---|---|
| ber. | C 36,90 | H 5,28 | N 10,12 | Eu 27,46 |
| gef. | C 36,71 | H 5,41 | N 9,97 | Eu 27,31 |

e) Praseodym-Komplex von 10-(2-Hydroxy-propyl)-1,4,7-tris-(carboxymethyl)-1,4,7,10-tetraazacyclododecan
Analog zu Beispiel 1c wurde 10-(2-Hydroxy-propyl)-1,4,7-tris-(carboxymethyl)-1,4,7,10-tetraazacyclododecan anstelle der Titelverbindung aus Beispiel 1a zur Komplexierung eingesetzt.
Ausbeute: 98 % d. Th. grünlicher Feststoff
Wassergehalt: 5,9 %

| Analyse (bezogen auf wasserfreie Substanz): | | | | |
|---|---|---|---|---|
| ber. | C 37,65 | H 5,39 | N 10,33 | Pr 25,98 |
| gef. | C 37,48 | H 5,48 | N 10,18 | Pr 25,81 |

### Beispiel 4

a) 10-(6-Hydroxy-2,2-dimethyl-1,3-dioxepan-5-yl)-1,4,7-tris(benzyloxycarbonyl)1,4,7,10-tetraazacyclododecan
40 g (126,4 mmol) 10-(6-Hydroxy-2,2-dimethyl-1,3-dioxepan-5-yl)-1,4,7,10-tetraazacyclododecan werden in 400 ml Methylenchlorid gelöst und 42,21 g (417,1 mmol) Triethylamin zugegeben. Bei 0°C tropft man innerhalb einer Stunde 71,16 g (417,1 mmol) Chlorameisensäurebenzylester hinzu und rührt eine Stunde bei 0°C, anschließend über Nacht bei Raumtemperatur. Man versetzt mit 500 ml Wasser und rührt 30 Minuten. Die organische Phase wird abgetrennt, über Magnesiumsulfat getrocknet und im Vakuum eingedampft. Der Rückstand wird an Kieselgel chromatographiert (Laufmittel=Methylenchlorid/Aceton 20:1).
Ausbeute: 79,05 g (87 % d. Th.) eines farblosen Öls

| Analyse | | | |
|---|---|---|---|
| ber. | C 65,16 | H 7,01 | N 7,79 |
| gef. | C 65,08 | H 7,15 | N 7,66 |

b) 10-(1-(2,2-Dimethyl-1,3-dioxolan-4-yl)-2-hydroxyethyl)-1,4,7-tris(benzyloxycarbonyl)-1,4,7,10-tetraazacyclododecan
7,8 g (108,5 mmol) der Titelverbindung aus Beispiel 4a und 19,61 g (113,9 mmol) p-Toluolsulfonsäure werden in 800 ml Ethanol gelöst und 3 Stunden bei 70°C gerührt. Man dampft zur Trockne ein, nimmt den Rückstand mit 500 ml Toluol auf und dampft erneut zur Trockne ein. Dieser Rückstand wird mit 600 ml Toluol aufgenommen und 53,4 ml (434 mmol) 2,2-Dimethoxypropan zugegeben. Dann wird 2 Stunden bei 80°C gerührt. Die Lösung wird auf Raumtemperatur abgekühlt und 3 mal mit 400 ml 3 N Natronlauge ausgeschüttelt. Die organische Phase wird über Magnesiumsulfat getrocknet und im Vakuum zur Trockne eingedampft. Der Rückstand wird an Kieselgel chromatographiert (Laufmittel=Methylenchlorid/Aceton 15:1)
Ausbeute: 56,94 g (73 % d. Th.) eines farblosen Öls

| Analyse | | | |
|---|---|---|---|
| ber. | C 65,16 | H 7,01 | N 7,79 |
| gef. | C 64,95 | H 6,88 | N 7,68 |

c) 10-[1-(2,2-Dimethyl-1,3-dioxolan-4-yl)-2-methoxyethyl]-1,4,7-tris(benzyloxycarbonyl)-1,4,7,10-tetraazacyclododecan
Zu 55 g (76,5 mmol) der Titelverbindung aus Beispiel 4b in 400 ml Methylenchlorid gibt man 21,46 g (382,5 mmol) Kaliumhydroxid (feingepulvert), sowie 500 mg Tetrabutylammonium-hydrogensulfat. Innerhalb einer Stunde wird bei Raumtemperatur 12,61 g (100 mmol) Dimethylsulfat unter kräftigem Rühren zugetropft. Man rührt 12 Stunden bei Raumtemperatur. Es werden 400 ml Eiswasser zugesetzt und 15 Minuten gerührt. Die organische Phase wird abgetrennt, über Magnesiumsulfat getrocknet und im Vakuum eingedampft. Der Rückstand wird an Kieselgel chromatographiert (Laufmittel=Methylenchlorid/Aceton 20:1).
Ausbeute: 52,14 g (93 % d. Th.) eines farblosen Öls

| Analyse | | | |
|---|---|---|---|
| ber. | C 65,56 | H 7,15 | N 7,64 |
| gef. | C 65,38 | H 7,24 | N 7,55 |

d) 1-[1-(2,2-Dimethyl-1,3-dioxolan-4-yl)-2-methoxyethyl]-1,4,7,10-tetraazacyclododecan
51 g (69,6 mmol) der Titelverbindung aus Beispiel 4c werden in 600 ml Ethanol gelöst und 5 g Palladiumkatalysator (10 % Pd/C) zugegeben. Man hydriert über Nacht bei Raumtemperatur. Der Katalysator wird abfiltriert und das Filtrat im Vakuum zur Trockne eingedampft.
Ausbeute: 23 g (quantitativ) glasiger Feststoff

| Analyse | | | |
|---|---|---|---|
| ber. | C 58,15 | H 10,37 | N 16,95 |
| gef.: | C 58,02 | H 10,45 | N 16,87 |

e) 10-(1-Methoxymethyl-2,3-dihydroxypropyl)-1,4,7-tris(carboxymethyl)-1,4,7,10-tetraazacyclododecan
8,35 g (25,28 mmol) der Titelverbindung aus Beispiel 4d werden in 50 ml Wasser gelöst und 14,05 g (101,12 mmol) Bromessigsäure zugesetzt. Der pH-Wert wird mit 6 normaler Kalilauge auf 9,5 gebracht. Man erwärmt auf 60°C und hält den pH-Wert durch Zugabe von 6 n Kalilauge zwischen 9,5 - 10. Nach 24 Stunden Rühren bei 60°C kühlt man im Eisbad, stellt mit konzentrierter Salzsäure auf pH 1 ein und dampft im Vakuum zur Trockne ein. Der Rückstand wird in wenig Wasser gelöst und auf eine Kationenaustauschersäule (IR 120) gegeben. Nach Spülung mit Wasser wird der Ligand mit 0,5 normaler wäßriger Ammoniak-Lösung eluiert. Die Fraktionen werden eingedampft, das Ammoniumsalz mit wenig Wasser aufgenommen und über eine Anionenaustauschersäule (IRA 67) gegeben. Man wäscht zuerst mit Wasser und eluiert dann mit 0,5 normaler wäßriger Ameisensäure. Man dampft im Vakuum ein, löst den Rückstand in wenig heißem Methanol und gibt Aceton hinzu. Nach Kühlung im Eisbad kristallisiert die Titelverbindung aus.
Ausbeute: 6,94 g (53 % d. Th.) eines amorphen, hygroskopischen Pulvers
Wassergehalt: 10,3 %

| Analyse (bezogen auf wasserfreie Substanz): | | | |
|---|---|---|---|
| ber. | C 49,13 | H 7,81 | N 12,06 |
| gef. | C 49,02 | H 7,95 | N 11,87 |

f) Lanthan-Komplex von 10-(1-Methoxymethyl-2,3-dihydroxypropyl)-1,4,7-tris(carboxymethyl)-1,4,7,10-tetraazacyclododecan
5 g (10,76 mmol) der Titelverbindung aus Beispiel 4e werden in 50 ml Wasser gelöst und 1,75 g (5,38 mmol) Lanthanoxid zugegeben. Man rührt 3 Stunden bei 90°C. Die Lösung wird eine Stunde mit 2 ml saurem Ionenaustauscher (AMB 252c/H⁺-Form) und 2 ml schwach basischem Austauscher (IRA 67/OH⁻-Form) bei Raumtemperatur gerührt. Es wird vom Austauscher abfiltriert und das Filtrat gefriergetrocknet.
Ausbeute: 6,76 g (97 % d. Th.) eines glasigen Feststoffes
Wassergehalt: 6,1%

| Analyse (bezogen auf wasserfreie Substanz): | | | | |
|---|---|---|---|---|
| ber. | 38,01 | H 5,54 | N 9,33 | La 23,14 |
| gef. | 37,83 | H 5,67 | N 9,18 | La 23,02 |

g) Dysprosium-Komplex von 10-(1-Methoxymethyl-2,3-dihydroxypropyl)-1,4,7-tris(carboxymethyl)-1,4,7,10-tetraazacyclododecan
Analog zu Beispiel 4f wurde Dysprosiumoxid anstelle von Lanthanoxid eingesetzt.
Ausbeute: 93 % d. Th. eines glasigen Feststoffes
Wassergehalt: 8,1 %

| Analyse (bezogen auf wasserfreie Substanz): | | | | |
|---|---|---|---|---|
| ber. | C 36,57 | H 5,33 | N 8,98 | Dy 26,04 |
| gef. | C 36,41 | H 5,41 | N 8,77 | Dy 25,84 |

h) Praseodym-Komplex von 10-(1-Methoxymethyl-2,3-dihydroxypropyl)-1,4,7-tris(carboxymethyl)-1,4,7,10-tetraazacyclododecan
Analog zu Beispiel 1c wurde die Titelverbindung aus Beispiel 4e anstelle der Titelverbindung aus Beispiel 1a zur Komplexierung eingesetzt.
Ausbeute: 95 % d. Th. eines grünlich gefärbten Feststoffes
Wassergehalt: 8,3 %

| Analyse (bezogen auf wasserfreie Substanz): | | | | |
|---|---|---|---|---|
| ber. | C 37,88 | H 5,52 | N 9,30 | Pr 23,39 |
| gef. | C 37,69 | H 5,63 | N 9,13 | Pr 23,20 |

i) Europium-Komplex von 10-(1-Methoxymethyl-2,3-dihydroxypropyl)-1,4,7-tris(carboxymethyl)-1,4,7,10-tetraazacyclododecan
Analog zu Beispiel 4f wurde Europiumoxid anstelle von Lanthanoxid eingesetzt.
Ausbeute: 98 % d. Th. eines farblosen amorphen Pulvers
Wassergehalt: 7,4 %

| Analyse (bezogen auf wasserfreie Substanz): | | | | |
|---|---|---|---|---|
| ber. | C 37,20 | H 5,42 | N 9,13 | Eu 24,77 |
| gef. | C 37,01 | H 5,61 | N 9,04 | Eu 24,58 |

### Beispiel 5

a) 1,3-Dimethoxy-propyl-2-trifluormethansulfonat
Zu 20 g (166 mmol) 1,3-Dimethoxy-2-hydroxypropan und 16,8 g (166 mmol) Triethylamin in 400 ml Diethylether tropft man bei -20°C 46,96 g (166 mmol) Trifluormethansulfonsäureanhydrid und rührt 2 Stunden bei dieser Temperatur. Anschließend rührt man 2 Stunden bei 0°C. Man gießt in 300 ml 20 %iger aqu. Kochsalz-Lösung und rührt kräftig durch. Die organische Phase wird abgetrennt und 2 mal mit 200 ml 20 %iger aq. Kochsalz-Lösung extrahiert. Die organische Phase wird über Magnesiumsulfat getrocknet und im Vakuum eingedampft.
Ausbeute: 39,77 g (95 % d. Th.) eines farblosen Öles (~ 97 % Gehalt laut GC) (Man setzt ohne Reinigung in Beispiel 5b weiter um)

| Analyse: | | | | |
|---|---|---|---|---|
| ber. | C 28,57 | H 4,40 | F 26,60 | S 12,69 |
| gef. | C 28,81 | H 4,65 | F 26,30 | S 12,38 |

b) 10-[1,1-Di(methoxymethyl)-methyl]-1,4,7-tris(benzyloxycarbonyl)-1,4,7,10-tetraazacyclododecan
39 g (154,6 mmol) der Titelverbindung aus Beispiel 5a und 44,43 g (77,3 mmol) 1,4,7-tris-(Benzyloxycarbonyl)- 1,4,7,10-tetraazacyclododecan (hergestellt nach Chem. Soc., Perkin Trans. 1, 12:3329, 1991) und 85,14 g (616 mmol) Kaliumcarbonat in 800 ml Acetonitril werden 24 Stunden lang unter Rückfluß erhitzt. Man filtriert vom Feststoff ab und engt das Filtrat im Vakuum zur Trockne ein. Der Rückstand wird in 700 ml Methylenchlorid aufgenommen und 2 mal mit 300 ml Wasser extrahiert. Die organische Phase wird über Magnesiumsulfat getrocknet und im Vakuum eingedampft. Der Rückstand wird an Kieselgel chromatographiert (Laufmittel=Methylenchlorid/Methanol 20:1).
Ausbeute: 18,31 g (35 % d. Th. bezgl. auf Amin) eines farblosen Öls

| Analyse: | | | |
|---|---|---|---|
| ber. | C 65,66 | H 7,15 | N 8,28 |
| gef. | C 65,47 | H 7,28 | N 8,13 |

c) 1-[1,1-Di(methoxymethyl)-methyl]-1,4,7,10-tetraazacyclododecan
18 g (26,6 mmol) der Titelverbindung aus Beispiel 5b werden in 300 ml Ethanol gelöst und 4 g Palladium-Katalysator (10 % Pd/C) zugegeben. Man hydriert 12 Stunden bei Raumtemperatur. Der Katalysator wird abfiltriert und das Filtrat im Vakuum zur Trockne eingedampft.
Ausbeute: 7,3 g (quantitativ) eines gelblichen zähen Öls

| Analyse: | | | |
|---|---|---|---|
| ber. | C 56,90 | H 11,02 | N 20,42 |
| gef. | C 56,71 | H 11,10 | N 20,28 |

d) 10-[1-Di(methoxymethyl)-methyl]-1,4,7-tris(carboxymethyl)-1,4,7,10-tetraazacyclododecan
6,94 g (25,28 mmol) der Titelverbindung aus Beispiel 5c werden in 50 ml Wasser gelöst und 14,05 g (101,12 mmol) Bromessigsäure zugesetzt. Der pH-Wert wird mit 6 normaler Kalilauge auf 9,5 gebracht. Man erwärmt auf 60°C und hält den pH-Wert durch Zugabe von 6 n Kalilauge zwischen 9,5 - 10. Nach 24 Stunden Rühren bei 60°C kühlt man im Eisbad, stellt mit konzentrierter Salzsäure auf pH 1 ein und dampft im Vakuum zur Trockne ein. Der Rückstand wird in wenig Wasser gelöst und auf eine Kationenaustauschersäule (IR 120) gegeben. Nach Spülung mit Wasser wird der Ligand mit 0,5 normaler wäßriger Ammoniak-Lösung eluiert. Die Fraktionen werden eingedampft, das Ammoniumsalz mit wenig Wasser aufgenommen und über eine Anionenaustauschersäule (IRA 67) gegeben. Man wäscht zuerst mit Wasser und eluiert dann mit 0,5 normaler wäßriger Ameisensäure. Man dampft im Vakuum ein, löst den Rückstand in wenig heißem Ethanol und gibt Aceton hinzu. Nach Kühlung im Eisbad kristallisiert die Titelverbindung aus.
Ausbeute: 7,71 g (61 % d. Th.) eines hygroskopischen Feststoffes
Wassergehalt: 10,3 %

| Analyse (bezogen auf wasserfreie Substanz): | | | |
|---|---|---|---|
| ber. | C 50,88 | H 8,09 | N 12,49 |
| gef. | C 50,64 | H 8,20 | N 12,27 |

e) Lanthan-Komplex von 10-[1-Di(methoxymethyl)-methyl]-1,4,7-tris(carboxymethyl)1,4,7,10-tetraazacyclododecan
5 g (11,15 mmol) der Titelverbindung aus Beispiel 5d werden in 50 ml Wasser gelöst und 1,82 g (5,57 mmol) Lanthanoxid zugegeben. Man rührt 3 Stunden bei 90°C. Die Lösung wird eine Stunde mit 2 ml saurem Ionenaustauscher (AMB 252c/H⁺-Form) und 2 ml schwach basischem Austauscher (IRA 67/OH⁻-Form) bei Raumtemperatur gerührt. Es wird vom Austauscher abfiltriert und das Filtrat gefriergetrocknet.
Ausbeute: 6,73 g (96 % d. Th.) eines glasigen Feststoffes
Wassergehalt: 6,7 %

| Analyse (bezogen auf wasserfreie Substanz): | | | | |
|---|---|---|---|---|
| ber. | C 39,05 | H 5,69 | N 9,59 | La 23,77 |
| gef. | C 38,84 | H 5,78 | N 9,41 | La 23,60 |

f) Dysprosium-Komplex von 10-[1-Di(methoxymethyl)-methyl]-1,4,7-tris(carboxymethyl)-1,4,7,10-tetraazacyclododecan
Analog zu Beispiel 5e wurde Dysprosiumoxid anstelle von Lanthanoxid eingesetzt.
Ausbeute: 93 % d. Th. eines farblosen Feststoffes
Wassergehalt: 7,3 %

| Analyse (bezogen auf wasserfreie Substanz): | | | | |
|---|---|---|---|---|
| ber. | C 37,54 | H 5,47 | N 9,21 | Dy 26,73 |
| gef. | C 37,71 | H 5,55 | N 9,05 | Dy 26,54 |

g) Europium-Komplex von 10-[1-Di(methoxymethyl)-methyl]-1,4,7-tris(carboxymethyl)1,4,7,10-tetraazacyclododecan
Analog zu Beispiel 5e wurde Europiumoxid anstelle von Lanthanoxid eingesetzt.
Ausbeute: 95 % d. Th. eines farblosen amorphen Pulvers
Wassergehalt: 8,5 %

| Analyse (bezogen auf wasserfreie Substanz): | | | | |
|---|---|---|---|---|
| ber. | C 38,20 | H 5,57 | N 9,38 | Eu 25,43 |
| gef. | C 38,03 | H 5,68 | N 9,24 | Eu 25,28 |

h) Praseodym-Komplex von 10-[1-Di(methoxymethyl)-methyl]-1,4,7-tris(carboxymethyl)-1,4,7,10-tetraazacyclododecan
Analog zu Beispiel 1c wurde die Titelverbindung aus Beispiel 5d anstelle der Titelverbindung 1a zur Komplexierung eingesetzt.
Ausbeute: 96 % d. Th. eines grünen Feststoffes
Wassergehalt: 3,9 %

| Analyse (bezogen auf wasserfreie Substanz): | | | | |
|---|---|---|---|---|
| ber. | C 38,92 | H 5,67 | N 9,55 | Pr 24,03 |
| gef. | C 38,73 | H 5,75 | N 9,47 | Pr 23,84 |

### Beispiel 6

a) 10-(3-oxa-butyl)-1,4,7,-tris(carboxymethyl)-1,4,7,10-tetraazacyclododecan
3 g (8,66 mmol) 1,4,7-Tris(carboxymethyl)-1,4,7,10-tetraazacyclododecan werden in 50 ml Dimethylformamid 10 Stunden mit 1,57 g (11,29 mmol) 2-Methoxy-ethylbromid und 4,15 g Kaliumcarbonat bei 90°C gerührt. Man dampft im Vakuum ein und verteilt den Rückstand zwischen 100 ml Wasser und 50 ml Diethylether. Die wässrige Phase wird mit 5 N Salzsäure auf pH 2 eingestellt und zur Trockne gedampft. Den Rückstand erhitzt man mit 200 ml Methanol unter Rückfluß, filtriert vom Ungelösten ab, dampft im Vakuum zur Trockne, löst den Rückstand in 50 ml Wasser, stellt mit 5 N Salzsäure auf pH 2 und gibt die Lösung über eine Säule mit 200 ml Kationenaustauscher IRC 50. Man eluiert zunächst mit 0,5 l Wasser, die man verwirft. Anschließend wird mit 0,5 l 0,5 N Ammoniaklösung eluiert. Man dampft im Vakuum nahezu zur Trockne, versetzt mit 100 ml Wasser und gibt unter Rühren soviel Kationenaustauscher IRC 50 zu, bis ein pH-Wert von 3,5 erreicht ist. Die Lösung wird dann filtriert und lyophilisiert. Man erhält 2,49 g der Titelverbindung mit einem Wassergehalt von 4,30 %.

| Analyse (bezogen auf wasserfreie Substanz): | | | |
|---|---|---|---|
| ber. | C 50,48 | H 7,79 | N 13,85 |
| gef. | C 50,31 | H 7,91 | N 13,95 |

b) Dysprosium-Komplex von 10-(3-oxabutyl)-1,4,7-tris-(carboxymethyl)- 1,4,7,10-tetraazacyclododecan
2 g (4,95 mmol) wasserfreie Substanz) der Titelverbindung aus Beispiel 6a werden in 25 ml Wasser mit 922 mg Dysprosiumoxid 8 Stunden bei 85°C gerührt. Man kühlt auf Raumtemperatur und rührt die Lösung 1 Stunde mit 1 ml saurem Ionenaustauscher (AMB 252c, H⁺-Form) und 1 ml basischen Ionenaustauscher (IRA 67, OH⁻-Form). Man filtriert, lyophilisiert und erhält 2,54 g der Titelverbindung.
Wassergehalt: 5,1 %

| Analyse (bezogen auf wasserfreie Substanz): | | | | |
|---|---|---|---|---|
| ber. | C 36,20 | H 5,18 | Dy 28,81 | N 9,93 |
| gef. | C 36,77 | H 5,32 | Dy 28,66 | N 10,05 |

c) Praseodym-Komplex von 10-(3-oxabutyl)-1,4,7-tris-(carboxymethyl)-1,4,7,10-tetraazacyclododecan
2,5 g (6,18 mmol, wasserfreie Substanz) der Titelverbindung aus Beispiel 6a werden in 30 ml Wasser mit 1,019 g (3,09 mmol) Praseodymoxid 5 Stunden bei 85°C gerührt. Man kühlt auf Raumtemperatur und rührt die Lösung 1 Stunde mit 1,3 ml saurem Ionenaustauscher (AMB 252c, H⁺-Form) und 1,3 mal basischen Ionenaustauscher (IRA 67, OH⁻-Form). Man filtriert, lyophilisiert und erhält 3,10 g der Titelverbindung mit einem Wassergehalt von 4,05 % als hellgrünen Feststoff.

| Analyse (bezogen auf wasserfreie Substanz): | | | | |
|---|---|---|---|---|
| ber. | C 37,64 | H 5,39 | N 10,33 | Pr 25,98 |
| gef. | C 37,96 | H 5,49 | N 10,32 | Pr 25,69 |

d) Holmium-Komplex von 10-(3-oxabutyl)-1,4,7-tris(carboxymethyl)-1,4,7,10-tetraazacyclododecan
Analog zu Beispiel 6c wird Holmium(III)oxid anstelle von Praseodymoxid eingesetzt.
Ausbeute: 90 % d. Th. eines farblosen Feststoffs
Wassergehalt: 4,90 %

| Analyse (bezogen auf wasserfreie Substanz): | | | | |
|---|---|---|---|---|
| ber. | C 36,05 | H 5,16 | Ho 29,12 | N 9,89 |
| gef. | C 36,35 | H 5,41 | Ho 29,01 | N 9,97 |

e) Ytterbium-Komplex von 10-(3-oxabutyl)-1,4,7-tris-(carboxymethyl)-1,4,7,10-tetraazacyclododecan
Analog zu Beispiel 6c wird Ytterbium(III)oxid anstelle von Praseodymoxid eingesetzt.
Ausbeute: 90 % d. Th. eines farblosen Feststoffs
Wassergehalt: 5,3 %

| Analyse (bezogen auf wasserfreie Substanz): | | | | |
|---|---|---|---|---|
| ber. | C 35,54 | H 5,09 | N 9,75 | Yb 30,12 |
| gef. | C 35,80 | H 5,31 | N 9,80 | Yb 30,07 |

f) Samarium-Komplex von 10-(3-oxabutyl)-1,4,7-tris(carboxymethyl)-1,4,7,10-tetraazacyclododecan
Analog zu Beispiel 6c wird Samarium(III)oxid anstelle von Praseodymoxid eingesetzt.
Ausbeute: 93 % d. Th. eines farblosen Feststoffs
Wassergehalt: 5,00 %

| Analyse (bezogen auf wasserfreie Substanz): | | | | |
|---|---|---|---|---|
| ber. | C 37,00 | H 5,30 | N 10,15 | Sm 27,24 |
| gef. | C 37,32 | H 5,52 | N 10,09 | Sm 27,02 |

g) Gadolinium-Komplex von 10-(3-oxabutyl)-1,4,7-tris-(carboxymethyl)-1,4,7,10-tetraazacyclododecan
Analog zu Beispiel 6c wird Gadoliumoxid anstelle von Praseodymoxid verwendet.
Ausbeute: 91 % d. Th. eines farblosen Feststoffs
Wassergehalt: 5,05 %

| Analyse (bezogen auf wasserfreie Substanz): | | | | |
|---|---|---|---|---|
| ber. | C 36,54 | H 5,23 | Gd 28,14 | N 10,02 |
| gef. | C 37,02 | H 5,27 | Gd 27,88 | N 10,17 |

h) Europium-Komplex von 10-(3-oxabutyl)-1,4,7-tris-(carboxymethyl)-1,4,7,10-tetraazacyclododecan
Analog zu Beispiel 6c wird Europium(III)oxid anstelle von Praseodymoxid verwendet.
Ausbeute: 95 % d. Th. eines farblosen Feststoffs
Wassergehalt: 4,30 %

| Analyse (bezogen auf wasserfreie Substanz): | | | | |
|---|---|---|---|---|
| ber. | C 36,89 | H 5,28 | Eu 27,46 | N 10,12 |
| gef. | C 37,31 | H 5,40 | Eu 27,00 | N 10,22 |

### Beispiel 7

a) 10-(4,4-Dimethyl-3-oxa-pentyl)-1,4,7-tris-(carboxymethyl)-1,4,7,10-tetraazacyclododecan
5 g (14,43 mmol) 1,4,7,-Tris-(carboxymethyl)-1,4,7,10-tetraazacyclododecan werden in 75 ml Dimethylformamid 18 Stunden mit 3,14 g (17,35 mmol) (2-Bromethyl)-tert.-butylether und 6,50 g Kaliumcarbonat bei 95°C gerührt. Man dampft im Vakuum ein und löst den Rückstand in 100 ml Wasser, stellt den pH-Wert durch Zugabe von 12 N Salzsäure auf 2 ein und gibt die Lösung auf eine Säule von 320 ml Kationenaustauscher IR-120 (H⁺-Form). Man eluiert mit 2 l Wasser, die man verwirft, danach mit 2 l 0,5 N Ammoniaklösung. Diese Lösung dampft man im Vakuum ein, löst den Rückstand in 100 ml Wasser. Unter Rühren gibt man portionsweise soviel Kationenaustauscher IR-120 zu bis zu einem pH-Wert von 3,5. Man filtriert und lyophilisiert die Lösung und erhält 4,19 g der Titelverbindung als weißen Feststoff mit einem Wassergehalt von 6,3 %.

| Analyse (bezogen auf wasserfreie Substanz): | | | |
|---|---|---|---|
| ber. | C 53,79 | H 8,58 | N 12,54 |
| gef. | C 54,01 | H 8,49 | N 12,70 |

b) Dysprosium-Komplex von 10-(4,4-Dimethyl-3-oxa-pentyl)-1,4,7-tris(carboxymethyl)1,4,7,10-tetraazacyclododecan
2,5 g (5,60 mmol, wasserfreie Substanz) der Titelverbindung aus Beispiel 7a werden mit 1,044 g Dysprosiumoxid (2,80 mmol) in 30 ml Wasser 5 Stunden bei 90°C gerührt. Nach dem Abkühlen rührt man die Lösung 1 Stunde mit 1,2 ml saurem Ionenaustauscher (AMB 252 c, H⁺-Form) und 1,2 mal basischen Ionenaustauscher (IRA 67, OH⁻-Form). Man filtriert, lyophilisiert und erhält 2,55 g der Titelverbindung als Feststoff.
Wassergehalt: 4,35 %

| Analyse (bezogen auf wasserfreie Substanz): | | | | |
|---|---|---|---|---|
| ber. | C 39,63 | H 5,82 | Dy 26,81 | N 9,24 |
| gef. | C 39,83 | H 5,90 | Dy 26,60 | N 9,32 |

c) Praseodym-Komplex von 10-(4,4-Dimethyl-3-oxa-pentyl)-1,4,7-tris(carboxymethyl)1,4,7,10-tetraazacyclododecan
Analog zu Beispiel 7b wird Praseodym(III)oxid anstelle von Dysprosiumoxid eingesetzt.
Ausbeute: 72 % eines hellgrünen Feststoffs
Wassergehalt: 4,2 %

| Analyse (bezogen auf wasserfreie Substanz): | | | | |
|---|---|---|---|---|
| ber. | C 41,10 | H 6,04 | N 9,59 | Pr 24,11 |
| gef. | C 41,29 | H 6,30 | N 9,68 | Pr 23,95 |

### Beispiel 8

a) 1,4-Di[4,7,10-tris(benzyloxycarbonyl)-1,4,7,10-tetraazacyclododecan-1-yl]-2,3-dihydroxybutan
44,43 g (77,3 mmol)1,4,7-tris(Benzyloxycarbonyl)-1,4,7,10-tetraazacyclododecan (hergestellt nach Chem. Soc. Perkin Trans 1, 12: 3329, 1991) und 2,21 g (25,67 mmol) 1,2-3,4-Diepoxybutan in 200 ml n-Butanol werden 3 Tage unter Rückfluß erhitzt. Man engt im Vakuum zur Trockne ein und chromatographiert den Rückstand an Kieselgel (Laufmittel: Methylenchlorid/Hexan/Aceton = 20/10/1).
Ausbeute: 15,22 g (48 % der Theorie bezogen auf 1,2-3,4-Diepoxybutan) eines zähen Öls

| Analyse: | | | |
|---|---|---|---|
| ber. | C 66,11 | H 6,69 | N 9,07 |
| gef. | C 66,03 | H 6,80 | N 9,15 |

b) 1,4-Di[4,7,10-tris(benzyloxycarbonyl)-1,4,7,10-tetraazacyclododecan-1-yl]-2,3-dimethoxybutan
Zu 15 g (12,14 mmol) der Titelverbindung aus Beispiel 8a und 1,44 g (60 mmol) Natriumhydrid in 200 ml Dimethylformamid gibt man 8,52 g (60 mmol) Methyliodid und rührt 48 Stunden bei 60° C. Man gibt 1000 ml Wasser hinzu und extrahiert 2 mal mit 300 ml Essigsäureethylester. Die vereinigten organischen Phasen werden 2 mal mit 500 ml Wasser extrahiert, anschließend die organische Phase über Magnesiumsulfat getrocknet und im Vakuum zur Trockne eingedampft. Der Rückstand wird an Kieselgel gereinigt (Laufmittel: Methylenchlorid/Hexan/Aceton = 20/10/1).
Ausbeute: 13,65 g (89 % der Theorie) eines farblosen Öls

| Analyse: | | | |
|---|---|---|---|
| ber. | C 66,54 | H 6,86 | N 8,87 |
| gef. | C 66,39 | H 6,95 | N 8,71 |

c) 1,4-Di[1,4,7,10-tetraazacyclododecan-1-yl]-2,3-dimethoxybutan
13,5 g (10,68 mmol) der Titelverbindung aus Beispiel 8b werden in 300 ml Ethanol gelöst und 4 g Palladium-Katalysator (10 % Pd/C) zugegeben. Man hydriert 12 Stunden bei Raumtemperatur. Der Katalysator wird abfiltriert und das Filtrat im Vakuum zur Trockne eingedampft.
Ausbeute: 4,9 g (quantitativ) eines leicht cremefarbenen Feststoffes

| Analyse: | | | |
|---|---|---|---|
| ber. | C 57,61 | H 10,99 | N 24,43 |
| gef. | C 57,51 | H 10,93 | N 24,48 |

d) 1,4-Di[4,7,10-tris(carboxymethyl)-1,4,7,10-tetraazacyclododecan-1-yl]-2,3-dimethoxybutan
4,8 g (10,46 mmol) der Titelverbindung aus Beispiel 8c werden in 80 ml Wasser gelöst und 13,96 g (100,5 mmol) Bromessigsäure zugesetzt. Der pH-Wert wird mit 6 normaler Kalilauge auf 9,5 gebracht. Man erwärmt auf 60° C und erhält den pH-Wert durch Zugabe von 6 n Kalilauge zwischen 9,5-10. Nach 24 Stunden Rühren bei 60° C kühlt man im Eisbad, stellt mit konzentrierter Salzsäure auf pH 1 ein und dampft im Vakuum zur Trockne ein. Der Rückstand wird in wenig Wasser gelöst und auf eine Kationenaustauschersäule (IR 120) gegeben. Nach Spülung mit Wasser wird der Ligand mit 0,5 normaler wäßriger Ammoniak-Lösung eluiert. Die Fraktionen werden eingedampft, das Ammoniumsalz mit wenig Wasser aufgenommen und über eine Anionenaustauschersäule (IRA 67) gegeben. Man wäscht zuerst mit Wasser und eluiert dann mit 0,5 normaler wäßriger Ameisensäure. Man dampft im Vakuum ein, löst den Rückstand in wenig heißem Ethanol und gibt Aceton hinzu. Nach Kühlung im Eisbad kristallisiert die Titelverbindung aus.
Ausbeute: 4,77 g (51 % der Theorie) eines hygroskopischen Feststoffes
Wassergehalt: 9,7 %

| Analyse (bezogen auf wasserfreie Substanz): | | | |
|---|---|---|---|
| ber. | C 50,61 | H 7,74 | N 13,89 |
| gef. | C 50,50 | H 7,81 | N 13,78 |

e) Bis-Praseodym-Komplex von 1,4-Di[4,7,10-tris(carboxymethyl)-1,4,7,10-tetraazacyclododecan-1-yl]-2,3-dimethoxybutan
4,5 g (5,577 mmol) der Titelverbindung aus Beispiel 8d werden in 50 ml Wasser gelöst und 1,84 g (5,577 mmol) Praseodymoxid zugegeben. Man rührt 3 Stunden bei 90° C. Die Lösung wird eine Stunde mit 2 ml saurem Ionenaustauscher (AMB 252c/H⁺-Form) und 2 ml schwach basischem Austauscher (IRA 67/OH--Form) bei Raumtemperatur gerührt. Es wird vom Austauscher abfiltriert und das Filtrat gefriergetrocknet.
Ausbeute: 6,47 g (97 % der Theorie) eines glasigen Feststoffes
Wassergehalt: 6,3 %

| Analyse (bezogen auf wasserfreie Substanz): | | | | |
|---|---|---|---|---|
| ber. | C 37,72 | H 5,21 | N 10,35 | Pr 26,03 |
| gef. | C 37,65 | H 5,29 | N 10,24 | Pr 25,95 |

### Beispiel 9

a) 1,7-Di[4,7,10-tris(benzyloxycarbonyl)-1,4,7,10-tetraazacyclododecan-1-yl]-2,6-dihydroxy-4-oxaheptan
44,43 g (77,3 mmol) 1,4,7-tris-(Benzyloxycarbonyl)-1,4,7,10-tetraazocyclododecan (hergestellt nach Chem. Soc. Perkin Trans 1, 12: 3329, 1991) und 3,34 g (25,67 mmol) 1,2-6,7-Diepoxy-4-oxa-heptan in 200 ml n-Butanol werden 3 Tage unter Rückfluß erhitzt. Man engt im Vakuum zur Trockne ein und chromatographiert den Rückstand an Kieselgel (Laufmittel: Methylenchlorid/Hexan/Aceton = 20/10/1).
Ausbeute: 17,74 g (54 % der Theorie bezogen auf 1,2-6,7-Diepoxy-4-oxa-heptan) eines zähen Öls.

| Analyse: | | | |
|---|---|---|---|
| ber. | C 65,71 | H 6,77 | N 8,76 |
| gef. | C 65,65 | H 6,84 | N 8,70 |

b) 1,7-Di[4,7,10-tris(benzyloxycarbonyl) 1,4,7,10-tetraazacyclododecan-1-yl]-2,6-dimethoxy-4-oxaheptan
Zu 17,5 g (13,68 mmol) der Titelverbindung aus Beispiel 9a und 1,64 g (68 mmol) Natriumhydrid in 230 ml Dimethylformamid gibt man 9,65 g (68 mmol) Methyliodid und rührt 48 Stunden bei 60° C. Man gibt 1000 ml Wasser hinzu und extrahiert 2 mal mit 300 ml Essigsäureethylester. Die vereinigten organischen Phasen werden 2 mal mit 500 ml Wasser extrahiert, anschließend die organische Phase über Magnesiumsulfat getrocknet und im Vakuum zur Trockne eingedampft. Der Rückstand wird an Kieselgel gereinigt (Laufmittel: Methylenchlorid/Hexan/Aceton = 20/10/1).
Ausbeute: 15,74 g (88 % der Theorie) eines farblosen Öls

| Analyse: | | | |
|---|---|---|---|
| ber. | C 66,14 | H 6,94 | N 8,57 |
| gef. | C 64,07 | H 6,91 | N 8,61 |

c) 1,7-Di(1,4,7,10-tetraazacyclododecan-1-yl)-2,6-dimethoxy-4-oxaheptan
15,5 g (11,85) mmol) der Titelverbindung aus Beispiel 9b werden in 300 ml Ethanol gelöst und 4 g Palladium-Katalysator (10 % Pd/C) zugegeben. Man hydriert 12 Stunden bei Raumtemperatur. Der Katalysator wird abfiltriert und das Filtrat im Vakuum zur Trockne eingedampft.
Ausbeute: 5,96 g (quantitativ) eines leicht cremefarbenen Feststoffes

| Analyse: | | | |
|---|---|---|---|
| ber. | C 57,34 | H 10,83 | N 22,29 |
| gef. | C 57,28 | H 10,90 | N 22,19 |

d) 1,7-Di[4,7,10-tris(carboxymethyl)-1,4,7,10-tetraazacyclododecan-1-yl]-2,6-dimethoxy-4-oxaheptan
5,8 g (11,54 mmol) der Titelverbindung aus Beispiel 9 c werden in 100 ml Wasser gelöst und 16,03 g (115,4 mmol) Bromessigsäure zugesetzt. Der pH-Wert wird mit 6 normaler Kalilauge auf 9,5 gebracht. man erwärmt auf 60° C und hält den pH-Wert durch Zugabe von 6 n Kalilauge zwischen 9,5-10. Nach 24 Stunden Rühren bei 60° C kühlt man im Eisbad, stellt mit konzentrierter Salzsäure auf pH 1 ein und dampft im Vakuum zur Trockne ein. Der Rückstand wird in wenig Wasser gelöst und auf eine Kationenaustauschersäule (IR 120) gegeben. Nach Spülung mit Wasser wird der Ligand mit 0,5 normaler wäßriger Ammoniak-Lösung eluiert. Die Fraktionen werden eingedampft, das Ammoiumsalz mit wenig Wasser aufgenommen und über eine Anionenaustauschersäule (IRA 67) gegeben. Man wäscht zuerst mit Wasser und eluiert dann mit 0,5 normaler wäßriger Ameisensäure. Man dampft im Vakuum ein, löst den Rückstand in wenig heißem Ethanol und gibt Aceton hinzu. Nach Kühlung im Eisbad kristallisiert die Titelverbindung aus.
Ausbeute: 6,65 (61 % der Theorie) eines hygroskopischen Feststoffes
Wassergehalt: 10,1 %

| Analyse (bezogen auf wasserfreie Substanz): | | | |
|---|---|---|---|
| ber. | C 50,81 | H 7,82 | N 13,17 |
| gef. | C 50,74 | H 7,85 | N 13,05 |

e) Bis Europium-Komplex von 1,7-Di[4,7,10-tris(carboxymethyl)-1,4,7,10-tetraazacyclododecan-1-yl]-2,6-dimethoxy-4-oxaheptan
6,5 g (7,638 mmol) der Titelverbindung aus Beispiel 9d werden in 50 ml Wasser gelöst und 2,68 g (7,638 mmol) Europiumoxid zugegeben. Man rührt 3 Stunden bei 90° C. Die Lösung wird eine Stunde mit 2 ml saurem Ionenaustauscher (AMB 252c/H⁺-Form) und 2 ml schwach basischem Austauscher (IRA 67/OH⁻-Form) bei Raumtemperatur gerührt. Es wird vom Austauscher abfiltriert und das Filtrat gefriergetrocknet.
Ausbeute: 9,26 g (98 % der Theorie) eines glasigen Feststoffes.
Wassergehalt: 7,2 %

| Analyse (bezogen auf wasserfreie Substanz): | | | | |
|---|---|---|---|---|
| ber. | C 37,64 | H 5,26 | N 9,75 | Eu 26,45 |
| gef. | C 37,53 | H 5,30 | N 9,70 | Eu 26,35 |

### Beispiel 10

Für ausgewählte Komplexe wurden zur Bestimmung verschiedener für die Temperaturmessung bedeutsamer Parameter entsprechende in-vitro Messungen durchgeführt. Dazu wurde von dem jeweiligen Komplex eine 0,01 molare wässrige Lösung hergestellt und bei verschieden Temperaturen im Bereich zwischen 46 °C und 26°C vermessen. Das intensive Signal des Lösungsmittels (H₂O) wurde durch selektive Vorsättigung und durch die Verwendung von schmalbandigen Audiofiltern unterdrückt. Die Ergebnisse dieser Untersuchungen sind in der Fig. 3/3 zusammengefaßt.

### Beispiel 11

a) 4-(α,α,α-Tris(hydroxymethyl)-methyl)-1,7-bis(p-tolylsulfonyl)-1,4,7-triazaheptan
Zu 40 g (330 mmol) Tris(hydroxymethyl)-methylamin gelöst in einer Mischung aus Ethanol/Wasser/Acetonitril (200/50/200 ml) gibt man 143,3 g (726 mmol) Tosylaziridin zu und rührt 2 Tage bei 45 °C. Man dampft zur Trockne ein und chromatographiert den Rückstand an Kieselgel (Laufmittel: Methylenchlorid/Ethanol 15:1).
Ausbeute: 97 g (57 % d. Th.) eines glasigen Feststoffs

| Elementaranalyse: | | | |
|---|---|---|---|
| ber. | C 51,24 | H 6,45 | N 8,15 |
| gef. | C 51,41 | H 6,55 | N 8,02 |

b) 1-((α,α,α-Tris(hydroxymethyl)-methyl)-4,7,10-tris(p-tolylsulfonyl)-1,4,7,10-tetraazacyclododecan
95 g (184,2 mmol) der Titelverbindung aus Beispiel 11a) werden in 900 ml Dimethylformamid gelöst und 24,31 g (1013 mmol) Natriumhydrid zugegeben. Man rührt 2 Stunden bei Raumtemperatur, anschließend wird auf 100 °C erwärmt. In diese Lösung tropft man 104,6 g (184,2 mmol) N-Bis[2-(4-Methylphenylsulfonyloxy)-ethyl]-4-methyl-phenylsulfonamid gelöst in 700 ml Dimethylformamid über 3 Stunden zu und rührt dann über Nacht bei 100 °C. Man engt im Vakuum zur Trockne ein, nimmt den Rückstand in 2000 ml Wasser auf und extrahiert 5 mal mit je 300 ml Methylenchlorid. Die organische Phase wird über Magnesiumsulfat getrocknet und zur Trockne eingedampft. Der Rückstand wird an Kieselgel chromatographiert (Laufmittel= Methylenchlorid/Ethanol 20:1)
Ausbeute: 50,36 g (37 % d. Th.) eines cremefarbenen Feststoffs

| Elementaranalyse: | | | | |
|---|---|---|---|---|
| ber. | C 53,64 | H 6,27 | N 7,58 | S 13,02 |
| gef. | C 53,75 | H 6,38 | N 7,49 | S 12,87 |

c) 1-(α,α,α-Tris(methoxymethyl)-methyl)-4,7,10-tris(p-tolylsulfonyl)-1,4,7,10-tetraazacyclododecan
50 g (67,66 mmol) der Titelverbindung aus Beispiel 11 b) werden in 500 ml Dimethylformamid gelöst und 6,5 g (270,64 mmol) Natriumhydrid zugegeben. Man rührt 6 Stunden bei Raumtemperatur. Dann tropft man 38,4 g (270,64 mol) Jodmethan gelöst in 100 ml Dimethylformamid über 30 Minuten zu und rührt anschließend über Nacht bei 50 °C. Man gibt vorsichtig 2000 ml Eiswasser zu und extrahiert 3 mal mit 300 ml Ethylacetat. Die organische Phase wird 2 mal mit 10 %iger Kochsalzlösung ausgeschüttelt und anschließend über Magnesiumsulfat getrocknet. Nach Eindampfen der organsichen Phase im Vakuum wird der Rückstand an Kieselgel chromatographiert (Laufmittel= Methylenchlorid/Aceton 15:1).
Ausbeute: 47,56 g (90 % d. Th.) eines farblosen Feststoffs

| Elementaranalyse: | | | | |
|---|---|---|---|---|
| ber. | C 55,36 | H 6,71 | N 7,17 | S 18,44 |
| gef. | C 55,43 | H 6,80 | N 7,05 | S 18,30 |

d) 1-(α,α,α-Tris(methoxymethyl)-methyl)-1,4,7,10-tetraazacyclododecan
Zu 45 g (57,62 mmol) der Titelverbindung aus Beispiel 11c) in 1000 ml n-Butanol gibt man bei 100 °C portionsweise 39,75 g (1,73 mol) Natrium zu und rührt über Nacht bei dieser Temperatur. Man kühlt auf Raumtemperatur ab, und gibt 1000 ml 20 %ige Kochsalzlösung zu. Die organische Phase wird abgetrennt und im Vakuum zur Trockne eingedampft. Der Rückstand wird an Kieselgel chromatographiert (Laufmittel= Ethanol/25 % aqu. Ammoniaklösung 10:1).
Ausbeute: 12,29 g (67 % d. Th.) eines zartgelben Öls, das beim Stehenlassen erstarrt
Wassergehalt: 5,1 %

| Elementaranalyse (berechnet auf wasserfreie Substanz): | | | |
|---|---|---|---|
| ber. | C 56,57 | H 10,76 | N 17,59 |
| gef. | C 56,40 | H 10,85 | N 17,43 |

e) 1-(α,α,α-Tris(methoxymethyl)-methyl)-4,7,10-tris(carboxymethyl)-1,4,7,10-tetraazacyclododecan
12 g (37,68 mmol) der Titelverbindung aus Beispiel 11d) werden in 200 ml Wasser gelöst und 26,2 g (188,4 mmol) Bromessigsäure zugesetzt. Der pH-Wert wird mit 6 normaler Kalilauge auf 9,5 gebracht. Man erwärmt auf 60 °C und hält den pH-Wert durch Zugabe von 6n Kalilauge zwischen 9,5-10. Nach 24 Stunden Rühren bei 60 °C kühlt man im Eisbad, stellt mit konz. Salzsäure auf pH 1 und dampft zur Trockne ein. Der Rückstand wird auf eine Kationenaustauschersäule (IR 120/H⁺-Form) gegeben. Nach Spülung mit Wasser wird der Ligand mit 0,5 normaler wässriger Ammoniak-Lösung eluiert. Die Fraktionen werden eingedampft und über eine Anionenaustauschersäule (IRA 67/OH⁻-Form) gegeben. Man wäscht mit Wasser und eluiert dann mit 0,5 normaler Ameisensäure. Man dampft im Vakuum zur Trockne ein, löst den Rückstand in wenig heißem Methanol und gibt Aceton zu. Nach Kühlung im Eisbad kristallisiert die Titelverbindung aus.
Ausbeute: 13,17 g (71 % d. Th.) eines glasigen Feststoffs
Wassergehalt: 9,3 %

| Elementaranalyse (berechnet auf wasserfreie Substanz): | | | |
|---|---|---|---|
| ber. | C 51,21 | H 8,18 | N 11,37 |
| gef. | C 51,13 | H 8,31 | N 11,24 |

f) Praseodym-Komplex von 1-(α,α,α-Tris(methoxymethyl)-methyl)4,7,10-tris(carboxymethyl)- 1,4,7,10-tetraazacyclododecan
4 g (8,12 mol) der Titelverbindung aus Beispiel 11e) werden in 40 ml Wasser gelöst und 1,34 g (4,06 mol) Praseodymoxid zugegeben. Man rührt 3 Stunden bei 90 °C. Die Lösung wird eine Stunde mit 5 ml saurem Ionenaustauscher (AMB 252c/H⁺-Form) und 5 ml schwach basischem Austauscher (IRA 67/OH⁻-Form) bei Raumtemperatur gerührt. Es wird vom Austauscher abfiltriert und gefriergetrocknet.
Ausbeute: 5,07 g (99 % d. Th.) eines glasigen Feststoffs
Wassergehalt: 10,1 %

| Elementaranalyse (berechnet auf wasserfreie Substanz): | | | | |
|---|---|---|---|---|
| ber. | C 40,01 | H 5,92 | N 8,89 | Pr 22,35 |
| gef. | C 39,87 | H 6,03 | N 8,73 | Pr 22,21 |

g) Europium-Komplex von 1-(α,α,α-Tris(methoxymethyl)-methyl)-4,7,10-tris(carboxymethyl)-1,4,7,10-tetraazacyclododecan
4 g (8,12 mmol) der Titelverbindung aus Beispiel 11e) werden in 40 ml Wasser gelöst und 1,43 g (4,06 mol) Europiumoxid zugegeben. Man rührt 3 Stunden bei 90 °C. Die Lösung wird eine Stunde mit 5 ml saurem Ionenaustauscher (AMB 252c/H⁺-Form) und 5 ml schwach basischem Austauscher (IRA 67/OH⁻-Form) bei Raumtemperatur gerührt. Es wird vom Austauscher abfiltriert und gefriergetrocknet.
Ausbeute: 5,10 g (98 % d. Th.) eines glasigen Feststoffs
Wassergehalt 9,7 %

| Elementaranalyse (berechnet auf wasserfreie Substanz): | | | | |
|---|---|---|---|---|
| ber. | C 39,32 | H 5,81 | N 8,73 | Eu 23,69 |
| gef. | C 39,25 | H 5,96 | N 8,61 | Eu 23,55 |

h) Dysprosium-Komplex von 1-(α,α,α-Tris(methoxymethyl)-methyl)-4,7,10-tris(carboxymethyl)-1,4,7,10-tetraazacyclododecan
4 g (8,12 mmol) der Titelverbindung aus Beispiel 11e) werden in 40 ml Wasser gelöst und 1,43 g (4,06 mol) Dysprosiumoxid zugegeben. Man rührt 3 Stunden bei 90 °C. Die Lösung wird eine Stunde mit 5 ml saurem Ionenaustauscher (AMB 252c/H⁺-Form) und 5 ml schwach basischem Austauscher (IRA 67/OH⁻-Form) bei Raumtemperatur gerührt. Es wird vom Austauscher abfiltriert und gefriergetrocknet.
Ausbeute: 5,24 g (99 % d. Th.) eines glasigen Feststoffs
Wassergehalt 8,9 %

| Elementaranalyse (berechnet auf wasserfreie Substanz): | | | | |
|---|---|---|---|---|
| ber. | C 38,68 | H 5,72 | N 8,59 | Dy 24,92 |
| gef. | C 38,51 | H 5,83 | N 8,47 | Dy 24,81 |

### Beispiel 12

a) 10-(3,6-Dioxa-heptyl)-1,4,7-tris(carboxymethyl)-1,4,7,10-tetraazacyclododecan 3 g (8,66 mmol) 1,4,7-tris(carboxymethyl)-1,4,7,10-tetraazacyclododecan werden in 50 ml Dimethylformamid 10 Stunden mit 1,83 g (10 mmol) 1-Brom-3,6-dioxa-heptan, 100 mg Natriumjodid und 4 g Kaliumcarbonat bei 90°C gerührt. Man dampft im Vakuum ein und verteilt den Rückstand zwischen 100 ml Wasser und 50 ml Diethylether. Die wässrige Phase wird mit 5 N Salzsäure auf pH 2 eingestellt und zur Trockne gedampft. Den Rückstand erhitzt man mit 200 ml Methanol unter Rückfluß, filtriert vom Ungelösten ab, dampft im Vakuum zur Trockne, löst den Rückstand in 50 ml Wasser, stellt mit 5 N Salzsäure auf pH 2 und gibt die Lösung über eine Säule mit 200 ml Kationenaustauscher IRC 50. Man eluiert zunächst mit 0,5 l Wasser, die man verwirft. Anschließend wird mit 0,5 l 0,5 N Ammoniaklösung eluiert. Man dampft im Vakuum nahezu zur Trockne, versetzt mit 100 ml Wasser und gibt unter Rühren soviel Kationenaustauscher IRC 50 zu, bis ein pH-Wert von 3,5 erreicht ist. Die Lösung wird dann filtriert und lyophilisiert. Man erhält 2,95 g der Titelverbindung mit einem Wassergehalt von 3,5 %.

| Elementaranalyse (berechnet auf wasserfreie Substanz): | | | |
|---|---|---|---|
| ber. | C 50,88 | H 8,09 | N 12,49 |
| gef. | C 50,92 | H 7,81 | N 12,32 |

b) Praseodym-Komplex von 10-(3,6-Dioxa-heptyl)-1,4,7-tris(carboxymethyl)-1,4,7,10-tetraazacyclododecan
2,50 g (5,57 mmol, bezogen auf wasserfreie Substanz) der Titelverbindung aus Beispiel 12a) werden in 30 ml Wasser mit 919 mg Praseodymoxid 4 Stunden bei 85°C gerührt. Man kühlt auf Raumtemperatur, filtriert und rührt die Lösung eine Stunde mit 1,5 ml saurem Ionenaustauscher (AMB 252c, H⁺-Form) und 1,5 ml basischem Ionenaustauscher (IRA 67, OH⁻-Form). Man filtriert, lyophilisiert und erhält 2,85 g der hellgrünen Titelverbindung mit einem Wassergehalt von 5,05 %.

| Elementaranalyse (berechnet auf wasserfreie Substanz): | | | | |
|---|---|---|---|---|
| ber. | C 38,92 | H 5,67 | N 9,55 | Pr 24,03 |
| gef. | C 38,69 | H 5,91 | N 9,71 | Pr 23,89 |

c) Dysprosiumkomplex von 10-(3,6-Dioxa-heptyl)-1,4,7-tris-(carboxymethyl)-1,4,7,10-tetraazacyclododecan
Analog zu Beispiel 12b) wird Dysprosiumoxid an Stelle von Praseodymoxid eingesetzt. Man erhält 3 g der Titelverbindung mit einem Wassergehalt von 4,5 %.

| Elementaranalyse (berechnet auf wasserfreie Substanz): | | | | |
|---|---|---|---|---|
| ber. | C 37,53 | H 5,47 | Dy 26,73 | N 9,22 |
| gef. | C 37,71 | H 5,70 | Dy 26,59 | N 9,38 |

### Beispiel 13

### Ziel der Untersuchung:

In vivo Temperaturmessung am narkotisierten Tier nach i.v. Gabe der Verbindung hergestellt nach Beispiel 6 c) mit 1H-Magnetresonanzspektroskopie (MRS). Vergleich der Meßergebnisse mit den Meßwerten einer Vergleichsmessung mit einem Temperaturfühler.

### Vorgehen:

Geräte: Elektrischer Temperaturfühler OTD 85, Fa. Ellab

Kernspintomograph: SIS 85/310, Fa. SISCO. 2 Tesla Magnet, Fa. Oxford.

Für die ¹H-MRS wurde eine Oberflächenspule mit Ø 20 mm im Leberbereich der Tiere plaziert. Es wurden Spektren mit 3-facher Wasserunterdrückung und Anregung mit frequenzselektivem Gausspuls (4 ms) mit nt=1000 und TR=0,25 sec aufgenommen.
Tiere: Ratte Wistar Han, 150g n=3.
Substanz: Verbindung hergestellt nach Beispiel 6 c), wässrige Lösung, 500 mmol/L, pH 7,4.
Dosis: 0,75 bis 1 mmol/kg i.v.

Die Tiere wurden zu Beginn der Untersuchung narkotisiert (Rompun-Ketavet). Danach sank ihre Körpertemperatur deutlich ab. Die Tiere wurden im Kernspintomographen auf einem Wasserbett plaziert, das zunächst auf Raumtemperatur belassen wurde. Es wurde gewartet, bis sich eine konstante Temperatur eingestellt hatte, die rektal mit einem Temperaturfühler gemessen wurde. Danach wurde die erste Dosis der Verbindung 6 c) verabreicht und es wurden hintereinander 6 Spektren á 4,3 Minuten aufgenommen. Anschließend wurde die rektale Temperatur erneut gemessen und danach das Wasserbett auf 41 °C erwärmt und wieder gewartet bis sich im Tier eine konstante rektale Temperatur eingestellt hatte. Die rektale Temperatur stieg durch diese Erwärmung um etwa 2-3 °C an. Nach Gabe einer zweiten Dosis der Verbindung 6 c) (die erste Dosis war in der Zwischenzeit bereits renal eliminiert worden) wurden erneut 6 Spektren á 4,3 Minuten aufgenommen. Anschließend wurde die rektale Temperatur erneut gemessen.

Die Auswertung der Spektren erfolgte durch Bestimmung des chemical shifts des Meßsignals vom Wassersignal, das als Referenz diente. Die Umrechnung des chemical shifts des Meßsignals in die Temperatur erfolgte mittels einer Eichkurve, die in Rinderplasma bei einer Konzentration von 2,5 mmol/L der Verbindung 6 c) aufgenommen wurde und die in vivo Bedingungen möglichst genau wiederspiegelt.

### Ergebnis:

In der Tabelle sind die Ergebnisse der Messung der Körpertemperatur der drei untersuchten Tiere zusammengefaßt. Die erste Spalte gibt den Mittelwert ± Stdabw. der Messung mit dem Temperaturfühler vor und nach der Spektroskopie an. Die Meßwerte der 6 Spektren wurden gemittelt und ihre Differenz zu dem Ergebnis des Thermofühlers wird in der zweiten Spalte angegeben. Die Stdabw. der 6 ¹H-MRS Meßwerte sind in der dritten Spalte angegeben und dienen als Maß für die Reproduzierbarkeit des Meßverfahrens.

| | Temperaturfühler, rektal (n=2) [°C] | Meßunterschied zwischen ¹H-MRS und dem Temperaturfühler [°C] | Reproduzierbarkeit der ¹H-MRS (n=6) [°C] |
|---|---|---|---|
| Tier, Narkose 1 | 31.5 ± 0.0 | + 2.3 | ± 0.4 |
| 2 | 29.9 ± 0.7 | + 0.5 | ± 0.6 |
| 3 | 30.3 ± 0.4 | - 1.1 | ± 0.3 |
| Tier, Narkose 1 | 34.5 ± 0.1 | 0 | ± 0.2 |
| + erwärmt 2 | 31.6 ± 0.7 | + 1.4 | ± 0.4 |
| 3 | 33.3 ± 0.6 | - 0.6 | ± 0.3 |

Bei den narkotisierten Tieren war mit einer Ausnahme, eine gute Übereinstimmung (Abweichung < 1.5 °C) zwischen den Meßwerten des Temperaturfühlers und der ¹H-MRS zu finden. Dabei ist zu berücksichtigen, daß die mit den beiden Methoden gemessenen Temperaturen nicht notwendigerweise exakt übereinstimmen müssen, da sie in verschiedenen Körperregionen gemessen werden (Temperaturfühler rektal und Spektroskopie im Leberbereich). Die Reproduzierbarkeit der jeweils 6 Spektren in einem Tier war gut (ca. ± 0.5 °C). Die Dosis von 0,75 bis 1 mmol/kg ist bei einer Messung mit der Oberflächenspule (ca. 5 ml erfaßtes Meßvolumen) ausreichend um über einen Zeitraum von etwa 30-45 Minuten p.a. mehrere aussagekräftige Spektren aufzunehmen.

### Fazit:

Mit der Verbindung 6 c) kann ein Temperaturunterschied von 1-2 °C in einem begrenzten Volumen mit ausreichender Verläßlichkeit (± 0.5 °C) dargestellt werden. Die Meßdauer für ein einzelnes Spektrum beträgt etwa 5 Minuten.

### Literaturverzeichnis:

(1) R. Duerst et al., Rev. Sci. Instrum. **36** (1965) 1896,
   F. Conti, Rev. Sci. Instrum. **38** (1967) 128,
   A.L. Van Geet, Anal. Chem. **40** (1968) 2227,
   A.L. Van Geet, Anal. Chem. **42** (1970) 678,
   C. Ammann et al., J. Magn. Reson. **46** (1982) 319;
(2) D.R. Vidrin et al., Anal. Chem. **48** (1976) 1301;
(3) R.K. Gupta et al., J. Magn. Reson. **40** (1980) 587;
(4) J.T. Bailey et al., J. Magn. Reson. **37** (1980) 353;
(5) P.E. Peterson, Anal. Chem. **50** (1978) 298;
(6) B.A. Berkowitz et al., NMR in Biomedicine **5** (1992) 65;
(7) MJ. Foster et al., J. Magn. Reson. **65** (1985) 497;
(8) K. Roth, Magn. Reson. Chem. **25** (1987) 429
(9) EP 0 095 124.

## Patentansprüche

1. Verwendung von makrocyclischen Metallkomplexen bestehend aus mindestens einem Metallion eines Elementes der Ordnungszahlen 21-29, 42, 44 oder 57-70 und einem Komplexbildner der allgemeinen Formel I 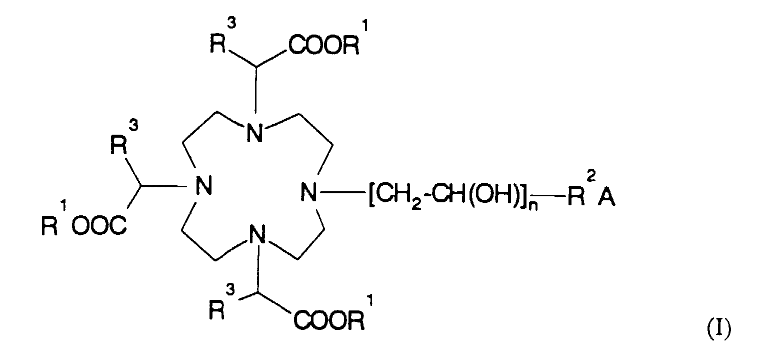 worin
n die Ziffern 0 oder 1 bedeutet
R¹ unabhängig voneinander für ein Wasserstoffatom oder ein Metallionenäquivalent,
R³ für ein Wasserstoffatom, eine geradkettige oder verzweigte C₁-C₁₀ Alkylgruppe, die gegebenenfalls durch 1-5 C₁-C₆-Alkoxygruppen, Hydroxy-C₁-C₆-alkylgruppen und/oder Hydroxygruppen substituiert ist,
R² eine geradkettige oder verzweigte C₁-C₁₀ Alkylengruppe, die gegebenenfalls durch 1 bis 5 Sauerstoffatome und oder Carbonylgruppen unterbrochen ist und/oder gegebenfalls durch 1 bis 5 Hydroxygruppen, C₁-C₆-Alkoxy-C₁-C₆-alkylgruppen, -OR⁴ -, -CO-NR⁵R⁶ -, -NR⁵R⁶ - und/oder -NR⁵-CO-R⁶- Reste substituiert ist, worin R⁴ für einen geradkettigen oder verzweigten C₁-C₄ Alkylrest steht und R⁵ R⁶ unabhängig voneinander die Bedeutung von R³ haben und
A für ein Wasserstoffatom oder einen zweiten makrocyclischen Rest der allgemeinen Formel II steht, 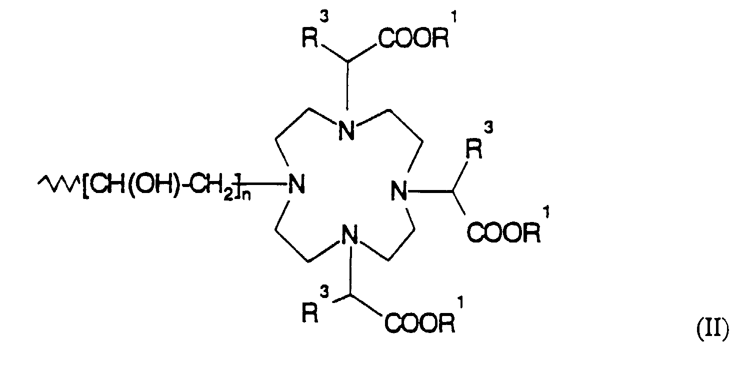
worin
n, R¹ und R³ die angegebenen Bedeutungen haben,
wobei freie nicht zur Komplexierung der Metallionen benötigten Carbonsäuregruppen gewünschtenfalls als Salz einer anorganischen oder organischen Base oder Aminosäure und/oder als Ester oder Amid vorliegen, wobei mindestens zwei Reste R¹ für ein Metallionenäquivalent stehen, als Temperatursonden in der NMR-Diagnostik.

2. Verwendung von makrocyclischen Metallkomplexen gemäß Anspruch 1 enthaltend als Rest -R²-A eine -CH₂CH₂OCH₃-, -CH₂CH₂O-C(CH₃)₃-, -CH₂-CH(OH)-CH₂OCH₃-, -CH₂-CH(OH)-CH₂O-CH(CH₃)₂-, -CH₂-CH(OH)-CH₂O-C(CH₃)₃-, -CH₂-CH(OH)-CH₃-, -CH(CH₂OCH₃)₂-, -CH(CH₂OCH₃)-CH(OH)CH₂OH-, -CH₂-CH₂-NH-CH₃-, -CH₂-CH₂-N(CH₃)₂-, -CH₂-CO-N(CH₃)₂-, -CH₂-CH₂-O-CH₂-CH₂-O-CH₃-, -C(CH₂OCH₃)₃-Gruppe.

3. Verwendung des Praseodym-Komplexes von 10-(3-oxabutyl)-1,4,7-tris(carboxymethyl)-1,4,7,10-tetraazacyclododecan zur Bestimmung der Gewebetemperatur.

4. Verwendung des Europium-Komplexes von 10-(3-oxabutyl)-1,4,7-tris(carboxymethyl)-1,4,7,10-tetraazacyclododecan zur Bestimmung der Gewebetemperatur.

## Claims

1. Use of macrocyclic metal complexes consisting of at least one metal ion of an element of atomic number 21-29, 42, 44 or 57-70 and a complexing agent of the general formula I 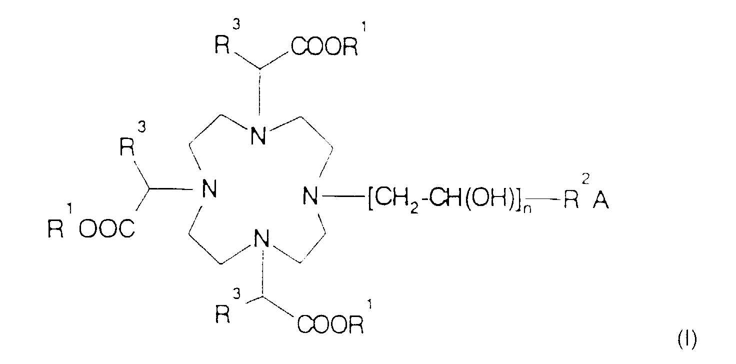 wherein
n represents the number 0 or 1,
each R¹ independently of the others represents a hydrogen atom or a metal ion equivalent,
R³ represents a hydrogen atom or a straight-chain or branched C₁-C₁₀alkyl group, which is optionally substituted by from 1 to 5 C₁-C₆alkoxy groups, hydroxy-C₁-C₆alkyl groups and/or hydroxy groups,
R² represents a straight-chain or branched C₁-C₁₀alkylene group, which is optionally interrupted by from 1 to 5 oxygen atoms and/or carbonyl groups and/or is optionally substituted by from 1 to 5 hydroxy groups, C₁-C₆alkoxy-C₁-C₆alkyl groups, -OR⁴, -CO-NR⁵R⁶, -NR⁵R⁶ and/or -NR⁵-CO-R⁶ radicals, wherein R⁴ represents a straight-chain or branched C₁-C₄alkyl radical and R⁵ and R⁶ each independently of the other have the same meanings as R³, and
A represents a hydrogen atom or a second macrocyclic radical of the general formula II 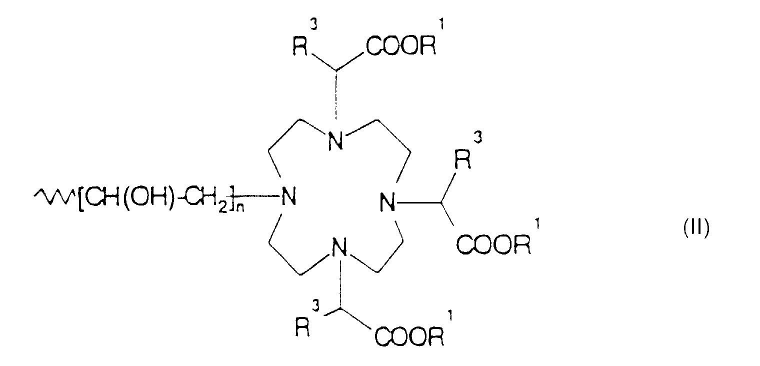
wherein
n, R¹ and R³ have the meanings indicated,
and wherein free carboxylic acid groups not required for complexing the metal ions are, if desired, in the form of the salt of an inorganic or organic base or amino acid and/or in ester or amide form, at least two of the radicals R¹ representing a metal ion equivalent,
as temperature probes in NMR diagnostics.

2. Use of macrocyclic metal complexes according to claim 1 containing as the radical -R²-A a group -CH₂CH₂OCH₃, -CH₂CH₂O-C(CH₃)₃, -CH₂-CH(OH)-CH₂OCH₃, -CH₂-CH(OH)-CH₂O-CH(CH₃)₂, -CH₂-CH(OH)-CH₂O-C(CH₃)₃, -CH₂-CH(OH)-CH₃, -CH(CH₂OCH₃)₂, -CH(CH₂OCH₃)-CH(OH)CH₂OH, -CH₂-CH₂-NH-CH₃, -CH₂-CH₂-N(CH₃)₂, -CH₂-CO-N(CH₃)₂, -CH₂-CH₂-O-CH₂-CH₂-O-CH₃ or -C(CH₂OCH₃)₃.

3. Use of the praseodymium complex of 10-(3-oxabutyl)-1,4,7-tris-(carboxymethyl)1,4,7,10-tetraazacyclododecane for determining the temperature of tissue.

4. Use of the europium complex of 10-(3-oxabutyl)-1,4,7-tris-(carboxymethyl)1,4,7,10-tetraazacyclododecane for determining the temperature of tissue.

## Revendications

1. Utilisation de complexes métalliques macrocycliques constitués d'au moins un ion métallique d'un élément des nombres atomiques 21-29, 42, 44 ou 57-70 et d'un complexant de la formule générale I 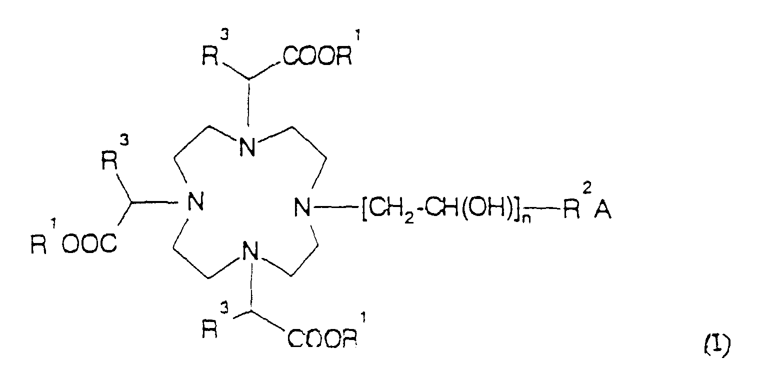 dans laquelle
n représente les chiffres 0 ou 1,
R¹ représente indépendamment l'un de l'autre un atome d'hydrogène ou un équivalent d'ion métallique,
R³ représente un atome d'hydrogène, un groupe alkyle en C₁-C₁₀ linéaire ou ramifié, qui est éventuellement substitué par 1 à 5 groupes alcoxy en C₁-C₆, groupes hydroxy-alkyle en C₁-C₆ et/ou groupes hydroxy,
R² représente un groupe alkylène en C₁-C₁₀ linéaire ou ramifié qui est éventuellement interrompu par 1 à 5 atomes d'oxygène et/ou groupes carbonyle et/ou est éventuellement substitué par 1 à 5 groupes hydroxy, groupes alcoxy en C₁-C₆-alkyle en C₁-C₆, radicaux -OR⁴, -CO-NR⁵R⁶, - NR⁵R⁶ et/ou -NR⁵-CO-R⁶, où R⁴ représente un radical alkyle en C₁-C₄ linéaire ou ramifié et R⁵ et R⁶ ont indépendamment l'un de l'autre la signification de R³, et
A représente un atome d'hydrogène ou un deuxième radical macrocyclique de la formule générale II 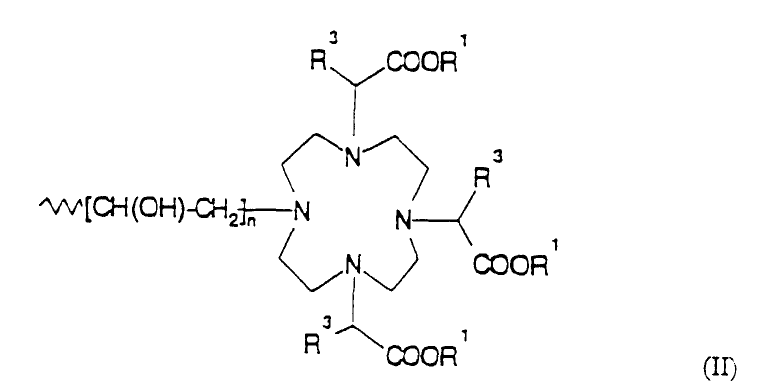 dans laquelle
n, R¹ et R³ ont la signification indiquée,
des groupes d'acide carboxylique libres, non nécessaires pour la complexation des ions métalliques, se présentant éventuellement sous la forme de sel d'une base inorganique ou organique ou d'un acide aminé et/ou sous la forme d'ester ou d'amide, au moins deux radicaux R¹ représentant un équivalent d'ion métallique,
à titre de sondes thermiques dans le diagnostic à RMN.

2. Utilisation de complexes métalliques macrocycliques suivant la revendication 1, contenant comme radical -R²-A, un groupe -CH₂CH₂OCH₃, -CH₂CH₂-O-C(CH₃)₃, -CH₂-CH(OH)-CH₂OCH₃, -CH₂-CH(OH)-CH₂O-CH(CH₃)₂, -CH₂-CH(OH)-CH₂O-C(CH₃)₃, -CH₂-CH(OH)-CH₃, -CH(CH₂OCH₃)₂, -CH(CH₂OCH₃)-CH(OH)CH₂OH, -CH₂-CH₂-NH-CH₃, -CH₂-CH₂-N(CH₃)₂, -CH₂-CO-N(CH₃)₂, -CH₂-CH₂-O- -CH₂-CH₂-O-CH₂, -C(CH₂OCH₃)₃.

3. Utilisation du complexe de praséodyme de 10-(3-oxabutyl)-1,4,7-tris-(carboxyméthyl)-1,4,7,10-tétraazacyclododécane, pour déterminer la température d'un tissu.

4. Utilisation du complexe d'europium de 10-(3-oxabutyl)-1,4,7-tris-(carboxyméthyl)-1,4,7,10-tétraazacyclododécane, pour la détermination de la température d'un tissu.
